# EUROPEAN PATENT APPLICATION

(11) **EP 4 089 084 A1**
(43) Date of publication of application: **16.11.2022**
(21) Application number: 21738753.9
(22) Date of filing: 07.01.2021
(51) Int. Cl.: C07D 403/04, C07C 39/19, C07D 401/10, A61P 25/04, A61K 31/352

(54) **CANNABIDIOL DERIVATIVE, AND PREPARATION METHOD THEREFOR AND MEDICAL USE THEREOF**

(30) Priority: 08.01.2020 CN 202010016914; 03.06.2020 CN 202010485399; 21.10.2020 CN 202011114139
(71) Applicant: Chengdu Baiyu Pharmaceutical Co., Ltd., Chengdu, Sichuan 611130 (CN)
(72) Inventor: ZHOU, Xibing, Chengdu, Sichuan 611130 (CN); ZHANG, Jing, Chengdu, Sichuan 611130 (CN); XU, Xuezhen, Chengdu, Sichuan 611130 (CN); WEI, Yonggang, Chengdu, Sichuan 611130 (CN); CHU, Hongzhu, Chengdu, Sichuan 611130 (CN); ZHAO, Fuqiang, Chengdu, Sichuan 611130 (CN); SU, Guizhuan, Chengdu, Sichuan 611130 (CN); WANG, Meiwei, Chengdu, Sichuan 611130 (CN); SUN, Yi, Chengdu, Sichuan 611130 (CN)
(74) Representative: Kador & Partner PartG mbB
(86) International application number: PCT/CN2021/070730
(87) International publication number: WO 2021/139741

(57) **Abstract**

The present invention relates to cannabidiol derivatives and medical use thereof, in particular to pyrimidine derivatives represented by general formula (I), or stereoisomers, solvates, metabolites, prodrugs, pharmaceutically acceptable salts or cocrystals thereof, wherein the definitions of substituents in general formula (I) are the same as those in the description

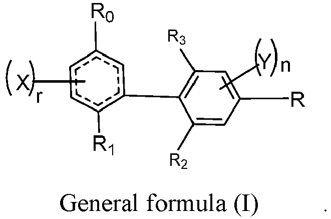

## Description

### TECHNICAL FIELD

The present application relates to cannabidiol derivatives, or stereoisomers, solvates, prodrugs, metabolites, pharmaceutically acceptable salts or cocrystals thereof, and pharmaceutical compositions comprising the cannabidiol derivatives, or stereoisomers, solvates, prodrugs, metabolites, pharmaceutically acceptable salts or cocrystals thereof, and use thereof in preparing medicament.

### BACKGROUND

As an annual herbaceous plant of Cannabis in Moraceae, hemp *(Cannabis sativa L.)* is originated in Central Asia and East Asia and is widely distributed in the United States, India, Brazil etc. Hemp has been used as a medicament for a long history, however, due to the addiction and hallucinogenic effect of hemp, its clinical application has been greatly limited. There are hundreds of different chemicals in hemp, and about 70 chemicals among them are named as cannabinoids, which mainly comprise cannabidiol (CBD), cannabinol (CBN), tetrahydrocannabinol (THC) and homologues thereof, among the cannabinoids, the content of cannabidiol (CBD) is the highest. Not only can CBD antagonize the mental activity induced by THC, it also has a wide range of therapeutic effects on neurological diseases comprising anxiety, schizophrenia, addiction, neurodegenerative diseases, neonatal hypoxic-ischemic encephalopathy and epilepsy. In addition, CBD also shows a promising application prospect in anti-tumor, anti-inflammatory, hepatoprotective, anti-pain, anti-anxiety, anti-insomnia, anti-convulsion, anti-vomiting, anti-spasm, anti-oxidation and neuroprotection.

### SUMMARY OF THE INVENTION

One object of the present application is to provide cannabidiol derivatives, or stereoisomers, solvates, prodrugs, metabolites, pharmaceutically acceptable salts, cocrystals or prodrugs thereof. Another object of the present application is to provide a pharmaceutical compositions comprising the cannabidiol derivatives, or stereoisomers, solvates, prodrugs, metabolites, pharmaceutically acceptable salts, cocrystals or prodrugs thereof. Yet another object is to provide use of the above compounds or the composition in preparing medicament.

One or more embodiments of the present application provide cannabidiol derivatives, which show good pharmacological activity, higher bioavailability (e.g., oral bioavailability), and longer half-life at the same time. After administration, the compounds according to the present invention can significantly reduce the production of inactive or toxic metabolites, prolong the drug action time, and show better pharmacokinetic characteristics, and reduced toxic and side effects.

One or more embodiments of the present application provide compounds represented by general formula (I), or stereoisomers, solvates, prodrugs, metabolites, pharmaceutically acceptable salts or cocrystals thereof: 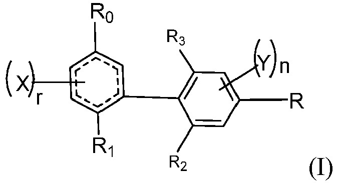 wherein,
R₀ is selected from the group consisting of methyl, -CH₂OH, -C(=O)OC₁₋₆ alkyl, -C(=O)NR^{b1}R^{b2} and carboxyl, and at least one hydrogen atom of R₀ is substituted by deuterium atom, and when R₀ is selected from the group consisting of -CD₂H and -CD₃, R is not -(CH₂)₄CH₃;
X is selected from the group consisting of hydrogen, deuterium, hydroxyl, C₁₋₆ alkyl and halogen;
R₁ is selected from the group consisting of C₁₋₆ alkyl, C₃₋₈ carbocyclic group and C₂₋₆ alkenyl, wherein the C₁₋₆ alkyl, the C₂₋₆ alkenyl and the C₃₋₈ carbocyclic group are optionally substituted by 1 to 3 substituents selected from the group consisting of halogen, hydroxyl, C₃₋₈ carbocyclic group and C₁₋₆ alkyl, and R₁ is optionally substituted by one or more deuterium atoms;
R₂ and R₃ are each independently selected from the group consisting of hydrogen, hydroxyl and C₁₋₆ alkoxy, R₂ and R₃ are each independently optionally substituted by one or more deuterium atoms, and at least one of R₂ and R₃ is not H;
r is selected from 0, 1, 2 and 3;
n is selected from 0, 1 and 2;
Y is selected from the group consisting of hydrogen, carboxyl, C₁₋₆ alkyl and halogen, and Y is optionally substituted by one or more deuterium atoms;
R is selected from the group consisting of C₁₋₁₂ alkyl, C₁₋₁₂ heteroalkyl, C₂₋₁₂ alkenyl, C₂₋₁₂ alkynyl, C₃₋₁₂ carbocyclic group, C₃₋₁₂ heterocyclic group, -C₁₋₆ alkylene-C₃₋₁₂ carbocyclic group, -C₁₋₆ alkylene-C₃₋₁₂ heterocyclic group, -NR^{b1}R^{b2}, -C₁₋₆ alkylene-C(=O)OC₁₋₆ alkyl and -C₁₋₆ alkylene-C(=O)NR^{b1}R^{b2}, wherein the C₁₋₁₂ alkyl, the C₁₋₁₂ heteroaklyl, the C₂₋₁₂ alkenyl, the C₂₋₁₂ alkynyl, the C₁₋₆ alkylene, the C₃₋₁₂ carbocyclic group and the C₃₋₁₂ heterocyclic group are optionally substituted with one or more substituents selected from the group consisting of hydroxyl, carboxyl, halogen, cyano, =O, C₁₋₆ alkyl, -NR^{b1}R^{b2}, C₃₋₁₂ carbocyclic group, C₃₋₁₂ heterocyclic group, C₂₋₆ alkenyl, C₂₋₆ alkynyl, -C(=O)OC₁₋₆ alkyl, -C(=O)C₁₋₆ alkyl, -C(=O)NR^{b1}R^{b2}, -S(=O)C₁₋₆ alkyl and -S(=O)₂C₁₋₆ alkyl, wherein as substituents, the C₁₋₆ alkyl, the C₃₋₁₂ carbocyclic group and the C₃₋₁₂ heteracyclic group as substituents are optionally further substituted with one or more substituents selected from the group consisting of =O, hydroxyl, carboxyl, halogen, cyano, -C(=O)OC₁₋₆ alkyl and -C(=O)C₁₋₆ alkyl, and R is optionally substituted by one or more deuterium atoms;
R^{b1} and R^{b2} are each independently selected from H, C₁₋₆ alkyl, C₃₋₁₂ carbocyclic group, C₃₋₁₂ heterocyclic group, -C(=O)R^{b3} and -C(=O)NR^{b4}R^{b5}, wherein the C₁₋₆ alkyl, the C₃₋₁₂ carbocyclic group or the C₃₋₁₂ heterocyclic group is optionally further substituted with one or more groups selected from the group consisting of hydroxy, deuterium atom, halogen, C₁₋₆ alkyl, C₁₋₆ alkoxy, C₆₋₁₂ aryl, C₅₋₁₂ hetroaryl, C₃₋₁₂ cycloalkyl, and C₃₋₁₂ heterocycloalkyl; or R^{b4} and R^{b5} together with N atom form a 3 to 12 membered heterocycle containing one or more heteroatoms selected from the group consisting of N, O and S, and R^{b1} and R^{b2} are optionally substituted by one or more deuterium atoms;
R^{b3} is selected from the group consisting of C₁₋₆ alkyl, C₁₋₆ alkoxy and C₆₋₁₂ aryl; and R^{b3} is optionally substituted by one or more deuterium atoms;
R^{b4} and R^{b5} are selected from the group consisting of H and C₁₋₆ alkyl, and R^{b4} and R^{b5} are optionally substituted by one or more deuterium atoms;
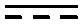 is a single bond or a double bond.

One or more embodiments of the present application provide the following compounds, or stereoisomers, solvates, prodrugs, metabolites, pharmaceutically acceptable salts or cocrystals thereof, wherein the compounds have the following structures:

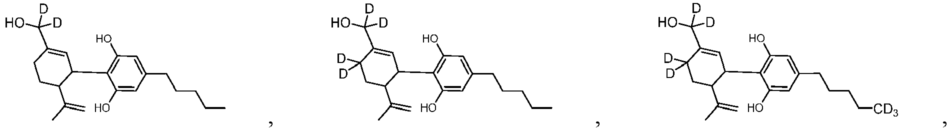

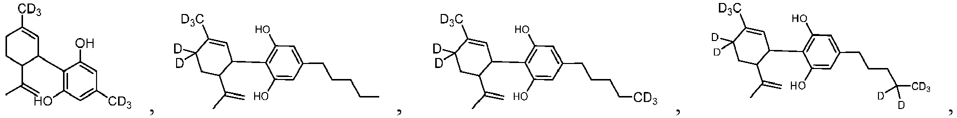

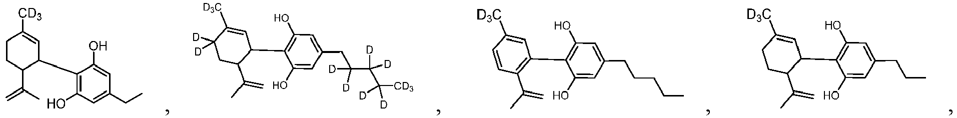

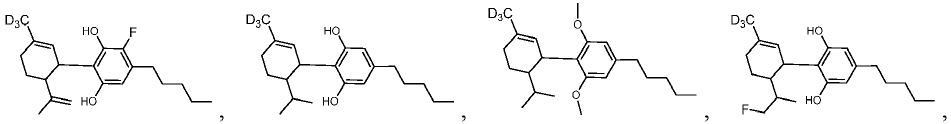

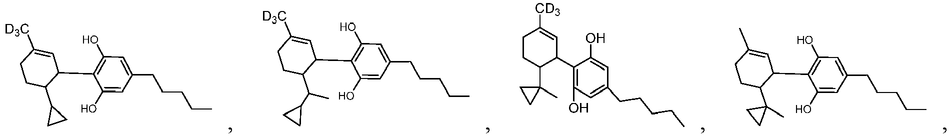

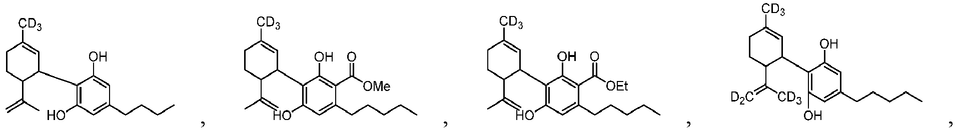

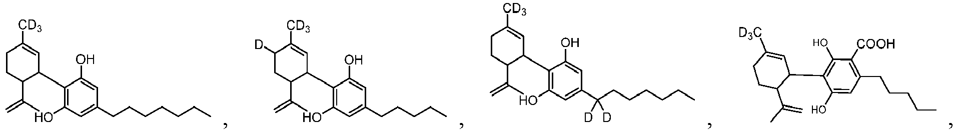

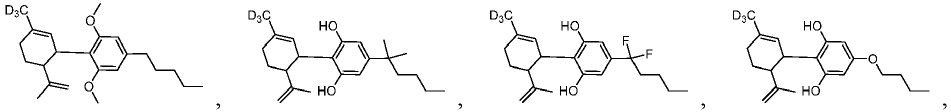

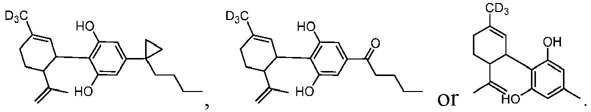

One or more embodiments of the present application provide an intermediate for preparing the compound represented by general formula (I), or stereoisomers, solvates, prodrugs, metabolites, pharmaceutically acceptable salts or cocrystals thereof, wherein the intermediate is selected from: 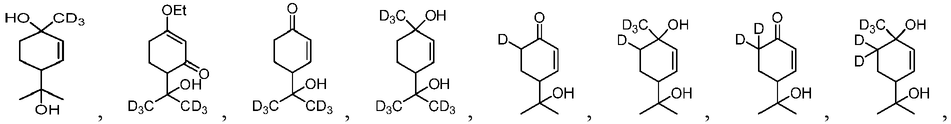 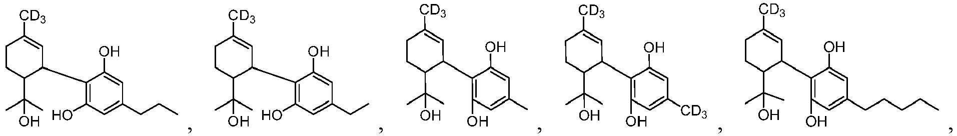 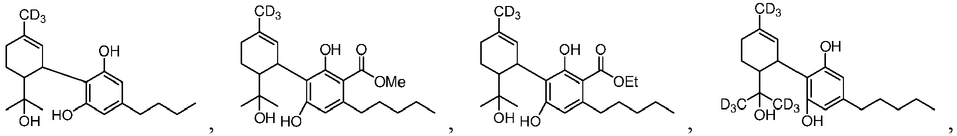 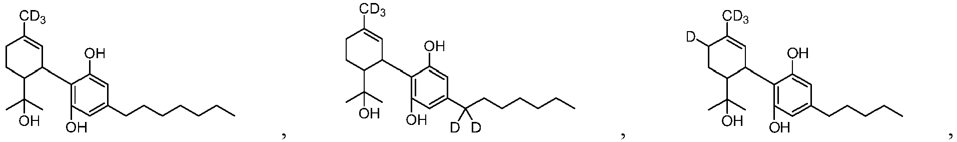 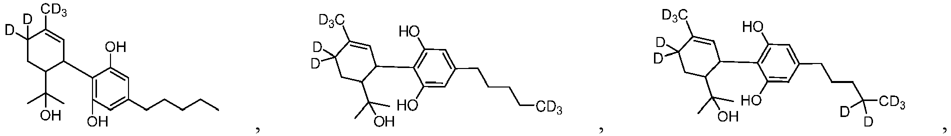 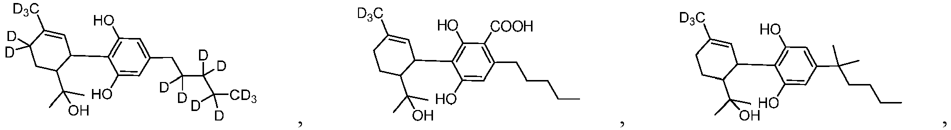 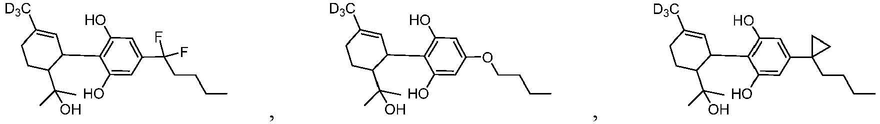 or

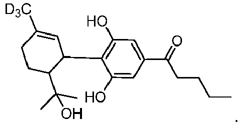

The present application also provides a method for preparing compound A or stereoisomers, deuterated products, solvates, prodrugs, metabolites, pharmaceutically acceptable salts or cocrystals, which comprises steps of: 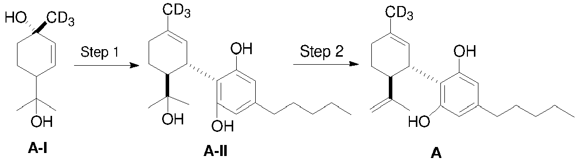
Step 1: reacting compound A-I, 3,5-dihydroxyamylbenzene and a protonic acid in an organic solvent to obtain compound A-II;
Step 2: subjecting compound A-II to a dehydration reaction in an organic solvent to obtain compound A.

The organic solvent can be determined by a person skilled in the art according to the common knowledge in the art.

One or more embodiments of the present application provide a pharmaceutical composition comprising:
(1) the compound according to the present application, or stereoisomers, solvates, prodrugs, metabolites, pharmaceutically acceptable salts cocrystals, or prodrugs thereof;
(2) optional one or more other active ingredients; and
(3) a pharmaceutically acceptable carrier and/or excipient.

In one or more embodiments of the present application, the other active ingredient is one or more selected from the group consisting of ginkgolides, antineoplastic agents, anticoagulants, antiepileptic agents, antidepressants, anxiolytics, hypnotics, analgesics and anesthetics, or stereoisomers, hydrates, metabolites, solvates, pharmaceutically acceptable salts or cocrytals thereof.

In one or more embodiments of the present application, the ginkgolide is one of ginkgolide A, ginkgolide B, ginkgolide C, ginkgolide D, ginkgolide J, ginkgolide M, ginkgolide K, ginkgolide L, ginkgolide N, ginkgolide P, ginkgolide Q or combinations of two or more thereof in any ratio.

One or more embodiments of the present application provide use of the compounds according to the present application, or stereoisomers, solvates, metabolites, pharmaceutically acceptable salts or cocrystals thereof or pharmaceutical composition thereof in the preparation of a medicament for treating post-traumatic stress disorder, facial paralysis, stroke, migraine, coronary heart disease stable angina pectoris, cerebral infarction, thromboembolism, myocardial infarction, cardiac ischemia, coronary artery disease, hypertension, cerebral ischemia, sexual function improvement, spasm, acute and chronic pain, fibromyalgia, postoperative pain, cluster headache, tension headache, back pain, limb pain, osphyalgia, neck pain, neuropathic pain, cancer pain, trigeminal neuralgia, arthritic pain, inflammatory pain, Dravet syndrome, Lennox-Gastaut syndrome, Prader-Willi syndrome, Sturge-Weber syndrome, fragile X syndrome, anxiety, bipolar affective disorder, autism, general anxiety disorder, social anxiety disorder, epilepsy, Parkinson's disease, Alzheimer's disease, Huntington's disease, opioid abuse, alcoholism, nicotine addiction, anorexia, cachexia, chemotherapy-related nausea and vomiting, postoperative nausea and vomiting, amyotrophic lateral sclerosis (ALS), Friedreich ataxia, schizophrenia, obsessive-compulsive disorder, multiple sclerosis, depression, sleep disorder, spasm caused by multiple sclerosis,dysmyotonia, sleep apnea, paralytic dementia, hypomnesis or glioblastoma.

One or more embodiments of the present application provide the above compounds of the present application for use as a medicament.

One or more embodiments of the present application provide the above compound according to the present application, or stereoisomers, solvates, metabolites, pharmaceutically acceptable salts or cocrystals thereof used in a method for treating the following disease: post-traumatic stress disorder, facial paralysis, stroke, migraine, coronary heart disease stable angina pectoris, cerebral infarction, thromboembolism, myocardial infarction, cardiac ischemia, coronary artery disease, hypertension, cerebral ischemia, sexual function improvement, spasm, acute and chronic pain, fibromyalgia, postoperative pain, cluster headache, tension headache, back pain, limbs pain, osphyalgia, neck pain, neuropathic pain, cancer pain, trigeminal neuralgia, arthritic pain, inflammatory pain, Dravet syndrome, Lennox-Gastaut syndrome, Prader-Willi syndrome, Sturge-Weber syndrome, fragile X syndrome, anxiety, bipolar affective disorder, autism, general anxiety disorder, social anxiety disorder, epilepsy, Parkinson's disease, Alzheimer's disease, Huntington's disease, opioid abuse, alcoholism, nicotine addiction, anorexia, cachexia, chemotherapy-related nausea and vomiting, postoperative nausea and vomiting, amyotrophic lateral sclerosis (ALS), Friedreich ataxia, schizophrenia, obsessive-compulsive disorder, multiple sclerosis, depression, sleep disorder, spasm caused by multiple sclerosis, dysmyotonia, sleep apnea, paralytic dementia, hypomnesis or glioblastoma.

One or more embodiments of the present application provide a method for treating the following disease, comprising administering to a subject in need thereof the above compound according to the present application, or stereoisomers, solvates, metabolites, pharmaceutically acceptable salts or cocrystals thereof: post-traumatic stress disorder, facial paralysis, stroke, migraine, coronary heart disease stable angina pectoris, cerebral infarction, thromboembolism, myocardial infarction, cardiac ischemia, coronary artery disease, hypertension, cerebral ischemia, sexual function improvement, spasm, acute and chronic pain, fibromyalgia, postoperative pain, cluster headache, tension headache, back pain, limbs pain, osphyalgia, neck pain, neuropathic pain, cancer pain, trigeminal neuralgia, arthritic pain, inflammatory pain, Dravet syndrome, Lennox-Gastaut syndrome, Prader-Willi syndrome, Sturge-Weber syndrome, fragile X syndrome, anxiety, bipolar affective disorder, autism, general anxiety disorder, social anxiety disorder, epilepsy, Parkinson's disease, Alzheimer's disease, Huntington's disease, opioid abuse, alcoholism, nicotine addiction, anorexia, cachexia, chemotherapy-related nausea and vomiting, postoperative nausea and vomiting, amyotrophic lateral sclerosis (ALS), Friedreich ataxia, schizophrenia, obsessive-compulsive disorder, multiple sclerosis, depression, sleep disorder, spasm caused by multiple sclerosis, dysmyotonia, sleep apnea, paralytic dementia, hypomnesis or glioblastoma.

Unless stated otherwise, the terms used in the description and claims have the following meanings.

Carbon, hydrogen, oxygen, sulfur, nitrogen or F, Cl, Br and I involved in the groups and compounds of the present application all comprise their isotopes, and carbon, hydrogen, oxygen, sulfur or nitrogen involved in the groups and compounds of the present application are optionally further replaced by one or more corresponding isotopes, wherein carbon isotopes comprise ¹²C, ¹³C and ¹⁴C, and hydrogen isotopes comprise protium (H), deuterium (D, also referred as heavy hydrogen), tritium (T, also referred as superheavy hydrogen); oxygen isotopes comprise ¹⁶O, ¹⁷O and ¹⁸O, sulfur isotopes comprise ³²S, ³³S, ³⁴ S and ³⁶S, nitrogen isotopes comprise ¹⁴N and ¹⁵N, fluorine isotopes comprise ¹⁷F and ¹⁹F, chlorine isotopes comprise ³⁵Cl and ³⁷Cl, and bromine isotopes comprise ⁷⁹Br and ⁸¹Br.

"Hydrocarbyl" refers to a group containing only carbon and hydrogen atoms.

"Alkyl" refers to a linear or branched saturated aliphatic hydrocarbyl having 1 to 20 carbon atoms, preferably an alkyl group having 1 to 8 (for example, 1, 2, 3, 4, 5, 6, 7, 8) carbon atoms, more preferably an alkyl group having 1 to 6 carbon atoms, and further preferably an alkyl group having 1 to 4 carbon atoms. Non-limiting examples thereof comprise methyl, ethyl, n-propyl, isopropyl, n-butyl, sec-butyl, neobutyl, tert-butyl, n-pentyl, isopentyl, neopentyl, n-hexyl and various branched isomers thereof; when the alkyl is substituted, the alkyl can be optionally further substituted by one or more substituents.

"Heteroalkyl" refers to a group in which at least one C atom of an alkyl is replaced by O, S, N or P atom. Non-limiting examples thereof comprise thiomethyl, thioethyl, thio n-propyl, thioisopropyl, thio n-butyl, thio sec-butyl and thio tert-butyl. The definition of alkyl is the same as that of the "alkyl" mentioned above.

"Alkoxyl" refers to a group in which at least one C atom of an alkyl is replaced by an oxygen atom. Non-limiting examples thereof comprise methoxy, ethoxy, n-propoxy, isopropoxy, n-butoxy, sec-butoxy, tert-butoxy, n-pentoxy, n-hexyloxy, cyclopropyloxy and cyclobutyloxy. The definition of alkyl is the same as that of the "alkyl" mentioned above.

"Alkenyl" refers to a straight or branched unsaturated aliphatic hydrocarbyl containing 1 to 10 (e.g., 1, 2, 3, 4, 5, 6, 7, 8, 9, 10) carbon-carbon double bonds and consisting of 2 to 20 carbon atoms, preferably 2 to 12 (e.g., 2, 3, 4, 5, 6, 7, 8, 9, 10, 11, 12) carbon atoms, more preferably 2 to 8 carbon atoms, even more preferably 2 to 6 carbon atoms. Non-limiting examples thereof comprise vinyl, propylen-2-yl, buten-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl and undecen-3-yl. The alkenyl may optionally be further substituted by one or more substituents.

"Alkynyl" refers to a straight or branched unsaturated aliphatic hydrocarbonyl containing 1 to 10 (e.g., 1, 2, 3, 4, 5, 6, 7, 8, 9 or 10) carbon-carbon triple bonds and consisting of 2 to 20 carbon atoms, preferably 2 to 12 (e.g., 2, 3, 4, 5, 6, 7, 8, 9, 10, 11 or 12) carbon atoms, more preferably 2 to 8 carbon atoms, even more preferably 2 to 6 carbon atoms. Non-limiting examples thereof comprise ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyne-3-yl, nonyn-3-yl, decyn-4-yl, undecyn-3-yl, and dodecyn-4-yl. The alkynyl may optionally be further substituted by one or more substituents.

"Aryl" refers to a substituted or unsubstituted aromatic ring, which can be 5 to 8 (e.g., 5, 6, 7, 8) membered monocyclic ring, a 5 to 12 (e.g., 5, 6, 7, 8, 9, 10, 11, 12) membered bicyclic ring, or a 10 to 15 (e.g., 10, 11, 12, 13, 14, 15) membered tricyclic system. The aryl can be a bridged ring or a spiro ring. Non-limiting examples thereof comprise phenyl and naphthyl. The aryl may optionally be further substituted by one or more substituents.

"Heteroaryl" refers to a substituted or unsubstituted aromatic ring, which can be a 3 to 8 (e.g., 3, 4, 5, 6, 7, 8) membered monocyclic ring, a 5 to 12 (e.g., 5, 6, 7, 8, 9, 10, 11, 12) membered bicyclic ring, or 10 to 15 (e.g., 10, 11, 12, 13, 14, 15) membered tricyclic system and contains 1 to 6 (e.g., 1, 2, 3, 4, 5, 6) heteroatoms selected from the group consisting of N, O and S. The heteroaryl is preferably a 5- to 8-membered heteroaryl. Optionally substituted 1 to 4 (for example, 1, 2, 3, 4) N and S in ring of the heteroaryl can be oxidized to various oxidation states. The heteroaryl can be connected to heteroatoms or carbon atoms, and the heteroaryl can be bridged ring or spiro rings. Non-limiting examples thereof comprise pyridyl, furyl, thienyl, pyranyl, pyrrolyl, pyrimidinyl, pyrazinyl, pyridazinyl, imidazolyl, piperidinyl, benzimidazolyl, benzopyridyl and pyrrolopyridinyl. The heteroaryl may optionally be further substituted by one or more substituents.

"Carbocyclic group" or "carbocyclic ring" refers to saturated or unsaturated aromatic ring or non-aromatic ring. When it is an aromatic ring, its definition is the same as the above definition of "aryl"; when it is a non-aromatic ring, it can be a 3 to 10 (e.g., 3, 4, 5, 6, 7, 8, 9, 10) membered monocyclic ring, a 4 to 12 (e.g., 4, 5, 6, 7, 8, 9, 10, 11, 12) membered bicyclic ring from , or a 10 to 15 (e.g., 10, 11, 12, 13, 14, 15) membered tricyclic system. Non-limiting examples thereof comprise cyclopropyl, cyclobutyl, cyclopentyl, 1-cyclopenten-1-yl, 1-cyclopenten-2-yl, 1-cyclopenten-3-yl, cyclohexyl, 1-cyclohexen-2-yl, 1-cyclohexen-3-yl, cyclohexenyl, cyclohexadienyl, cycloheptyl, cyclooctyl, cyclononyl, cyclodecyl, cycloundecyl, cyclododecyl, 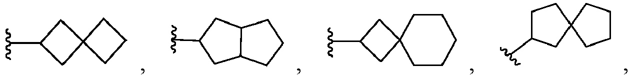 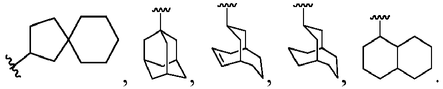 The "carbocyclic group" or "carbocyclic ring" may optionally be further substituted by one or more substituents.

"Heterocyclic group" or "heterocyclic ring" refers to a saturated or unsaturated aromatic heterocyclic ring or non-aromatic heterocyclic ring. When it is an aromatic heterocyclic ring, its definition is the same as that of "heteroaryl" above. When it is a non-aromatic heterocyclic ring, it can be a 3 to 10 (e.g., 3, 4, 5, 6, 7, 8, 9, 10) membered monocyclic ring, a 4 to 12 (e.g., 4, 5, 6, 7, 8, 9, 10, 11, 12) membered bicyclic ring, or a 10 to 15 (e.g., 10, 11, 12, 13, 14, 15) membered tricyclic system and contains 1 to 4 heteroatoms selected from the group consisting of N, O or S. It is preferably a 3 to 8 membered heterocyclic group. Optionally substituted 1 to 4 (e.g., 1, 2, 3, 4) N or S in the ring of "heterocyclic group" or "heterocyclic ring" can be oxidized into various oxidation states; "heterocyclic group" or "heterocyclic ring" can be connected to a heteroatom or a carbon atom; "heterocyclic group" or "heterocyclic ring" can be a bridged ring or a spiro ring. Non-limiting examples thereof comprise oxiranyl, epoxypropyl, aziridinyl, oxetanyl, azetidinyl, thietanyl , 1,3-dioxolanyl, 1,4-dioxolanyl, 1,3-dioxanyl, azepanyl, oxepanyl, thiepanyl, oxazepinyl, diazepinyl, thiazepinyl, pyridyl, piperidinyl, homopiperidinyl, furyl, thienyl, pyranyl, N-alkylpyrrolyl, pyrimidinyl, pyridinyl, pyridazinyl, piperazinyl, homopiperazinyl, imidazolyl, piperidinyl, morpholinyl, thiomorpholinyl, thioxanyl, 1,3-dithianyl, dihydrofuranyl, dithiolanyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuranyl, 2-pyrrolinyl, 3-pyrrolinyl, indolinyl, 2H-pyranyl, 4H-pyranyl , dioxanyl, 1,3-dioxolanyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3H-indolylquinazinyl, N-pyridylurea, 1,1-dioxothiomorpholinyl, azabicyclo[3.2.1]octyl, 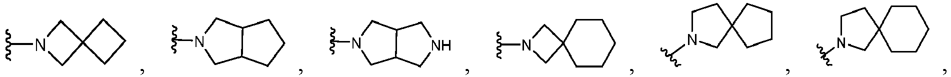 azabicyclo [5.2.0] nonyl, oxatricyclo[5.3.1.1]dodecyl, azaadamantyl and oxaspiro[3.3]heptyl. The "heterocyclic group" or "heterocyclic ring" may be optionally further substituted by 0 or more substituents.

"Cycloalkyl" refers to a saturated cyclic hydrocarbyl, which can be a 3 to 10 (e.g., 3, 4, 5, 6, 7, 8, 9, 10) membered monocyclic ring, a 4 to 12 (e.g., 4 , 5, 6, 7, 8, 9, 10, 11, 12) membered bicyclic ring or 10 to 20 (e.g., 4, 5, 6, 7, 8, 9, 10, 11, 12) membered polycyclic system. There are preferably 3 to 10, more preferably 3 to 8 ring carbon atoms. Non-limiting examples of "cycloalkyl" comprise cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl and cycloheptatrienyl, etc. When the cycloalkyl is substituted, it can be optionally further substituted by one or more substituents.

"Heterocycloalkyl" refers to a substituted or unsubstituted saturated non-aromatic ring group, which can be 3 to 8 (e.g., 3, 4, 5, 6, 7, 8) membered monocyclic ring, 4 to 12 (e.g., 4, 5, 6, 7, 8, 9, 10, 11, 12) membered bicyclic ring or 10 to 15 (e.g., 10, 11, 12, 13, 14, 15) membered tricyclic system, and contains 1, 2 or 3 heteroatoms selected from the group consisting of N, O and S. It is preferably a 3 to 8 membered heterocyclic group. Optionally substituted N or S in the ring of "heterocycloalkyl" can be oxidized to various oxidation states; "heterocycloalkyl" can be connected to a heteroatom or a carbon atom; and the "heterocycloalkyl" can be a bridged ring or a spiro ring. Non-limiting examples thereof comprise oxiranyl, aziridinyl, oxetanyl, azetidinyl, 1,3-dioxolanyl, 1,4-dioxolanyl, 1,3-dioxanyl, azepanyl, piperidinyl, piperidinyl, morpholinyl, thiomorpholinyl, 1,3-dithianyl, tetrahydrofuranyl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecyl, azaadamantyl and oxaspiro[3.3]heptyl.

When the above-mentioned "alkyl", "alkoxy", "alkenyl", "alkynyl", "aryl", "heteroaryl", "carbocyclic group", "carbocyclic ring", "heterocyclic group", "heterocyclic ring", "cycloalkyl", "heterocycloalkyl" or "heterocyclic group" are substituted, they may be optionally substituted with 0, 1, 2, 3, 4, 5, 6, 7, 8, 9 or 10 substituents selected from F, Cl, Br, I, hydroxyl, mercapto, nitro, cyano, amino, C₁₋₆ alkylamino, =O, C₁₋₆ alkyl, C₁₋₆ alkoxy, C₂₋₆ alkenyl, C₂₋₆ alkynyl, -NR^{q4}R^{q5}, =NR^{q6}, -C(=O)OC₁₋₆ alkyl, -OC(=O)C₁₋₆ alkyl, - C(=O)NR^{q4}R^{qs}, C₃₋₈ cycloalkyl, C₃₋₈ heterocycloalkyl, C₆₋₁₀ aryl, C₅₋₁₀ heteroaryl, -C(=O)OC₆₋₁₀ aryl, -OC(=O)C₆₋₁₀ aryl, -OC(=O)C₅₋₁₀ heteroaryl, -C(=O)OC₅₋₁₀ heteroaryl, -OC(=O)C₃₋₈ heterocycloalkyl, -C(=O)OC₃₋₈ heterocycloalkyl, -OC(=O)C₃₋₈ cycloalkyl, -C(=O)OC₃₋₈ cycloalkyl, -NHC(=O)C₃₋₈ heterocycloalkyl, -NHC(=O)C₆₋₁₀ aryl, -NHC(=O)C₅₋₁₀ heteroaryl, -NHC(=O)C₃₋₈cycloalkyl, -NHC(=O)C₃₋₈ heterocycloalkyl, -NHC(=O)C₂₋₆ alkenyl or - NHC(=O)C₂₋₆ alkynyl, wherein the substituents C₁₋₆ alkyl, C₁₋₆ alkoxy, C₂₋₆ alkenyl, C₂₋₆ alkynyl, C₃₋₈ cycloalkyl, C₃₋₈heterocycloalkyl, C₆₋₁₀ aryl, C₅₋₁₀ heteroaryl, -NHC(=O)C₆₋₁₀ aryl, -NHC(=O)C₅₋₁₀ heteroaryl, -NHC(=O)C₃₋₈ heterocycloalkyl or -NHC(=O)C₃₋₈ cycloalkyl can be optionally substituted with 1 to 3 substituents selected from OH, F, Cl, Br, I, C₁₋₆ alkyl, C₁. ₆ alkoxyl, -NR^{q4}R^{q5} or =O. R_{q1} is selected from C₁₋₆ alkyl, C₁₋₆alkoxyl or C₆₋₁₀ aryl; R^{q2} or R^{q3} is selected from H or C₁₋₆ alkyl; wherein, R^{q4} and R^{q5} are selected from H, C₁₋₆ alkyl, - NH(C=NR^{q1})NR^{q2} R^{q3}, -S(=O)₂NR^{q2}R^{q3}, -C(=O)R^{q1} or -C(=O)NR^{q2}R^{q3}, and the C₁₋₆ alkyl is optionally substituted with one or more substituents selected from OH, F, Cl, Br, I, C₁₋₆ alkyl, C₁₋₆ alkoxy, C₆₋₁₀ aryl, C₅₋₁₀ heteroaryl, C₃₋₈ cycloalkyl, or C₃₋₈ heterocycloalkyl; or R^{q4} and R^{q5} together with N atom form 3 to 8 membered heterocyclic ring containing one or more heteroatoms selected from N, O or S.

"Amino acid side chain" refers to a group other than amino and carboxyl groups in an amino acid molecule.

"Pharmaceutically acceptable salt" or "pharmaceutically acceptable salt thereof' means that the compound of the present application maintains the biological effectiveness and characteristics of the free acid or free base, and the salt can be obtained by reacting the free acid with a non-toxic inorganic base or organic base, or the salt can be obtained by reacting the free base with a non-toxic inorganic acid or organic acid.

"Pharmaceutical composition" refers to a mixture of one or more compounds according to the present application, pharmaceutically acceptable salts or prodrugs thereof and other chemical components, wherein "other chemical components" refer to pharmaceutically acceptable carriers, excipients and/or one or more other therapeutic agents.

"Carrier" refers to a material that does not cause obvious irritation to organisms and does not eliminate the biological activity and characteristics of the administered compound.

"Excipient" refers to an inert substance added to a pharmaceutical composition to facilitate the administration of compounds. Non-limiting examples comprise calcium carbonate, calcium phosphate, sugar, starch, cellulose derivatives (including microcrystalline cellulose), gelatin, vegetable oil, polyethylene glycols, diluents, granulating agents, lubricants, adhesives and disintegrating agents.

"Prodrug" refers to a compound which can be converted into the compound according to the present application having biological activity by metabolism in vivo. The prodrugs of the present application can be prepared by modifying amino or carboxyl groups in the compound of the present application, and the modification can be removed by conventional operation or in vivo to obtain the parent compound. When the prodrug of the present application is administered to a mammalian subject, the prodrug is cleaved to form free amino groups or carboxyl groups.

"Cocrystal" refers to the crystal formed by the combinations of active pharmaceutical ingredients (API) and cocrystal former (CCF) under the action of hydrogen bonds or other noncovalent bonds, in which the pure states of API and CCF are both solid at room temperature, and there is a fixed stoichiometric ratio among the components. Cocrystal is a multi-component crystal, which comprises binary cocrystal formed between two neutral solids and multicomponent cocrystal formed by neutral solids and salts or solvates.

"Stereoisomers" refer to isomers produced by different spatial arrangement of atoms in molecules, including cis-trans isomers, enantiomers and conformational isomers.

"Optional", "optionally", "selective" or "selectively" means that the event or condition described subsequently can, but does not necessarily, occur, and the description comprises the situation in which the event or condition occurs and the situation in which the event or condition does not occur. For example, "heterocyclic group optionally substituted by alkyl" means that the alkyl group may, but does not necessarily exist, and the description comprises the situation where the heterocyclic group is substituted by alkyl, and the situation where the heterocyclic group is not substituted by alkyl.

### SPECIFIC EMBODIMENTS

The following Examples will illustrate the technical solution of the present application in detail, and the scope of the present application comprises, but is not limited these examples.

The structure of the compounds were determined by nuclear magnetic resonance (NMR) or/and mass spectrometry (MS). NMR shift (δ) was given in units of 10⁻⁶ (ppm). The NMR test was conducted by using nuclear magnetic instrument (Bruker Avance III 400 and Bruker Avance 300), and the solvent for the test was deuterated dimethyl sulfoxide (DMSO-d₆), deuterated chloroform (CDCl₃), deuterated methanol (CD₃OD), and the internal standard was tetramethylsilane (TMS).

MS test was conducted on Agilent 6120B(ESI) and Agilent 6120B(APCI);

HPLC test was conducted on Agilent 1260DAD high pressure liquid chromatograph (zorbax sb-C18 100× 4.6 mm, 3.5 µ m).

HSGF254 silica gel plate from Yantai Huanghai or Qingdao GF254 silica gel plate was used for thin layer chromatography (TLC), and the size of the silica gel plate for thin layer chromatography (TLC) was 0.15 mm to 0.20 mm, and the size of the silica gel plate for thin layer chromatography separation and purification products was 0.4 mm to 0.5 mm.

Silica gel of 200-300 mesh by Yantai Huanghai was generally used as carrier of the column chromatography.

The known starting materials in the present application can be synthesized by using the methods known in the field, or can be purchased from Titan Technology company, Annaiji Chemical company, Shanghai DEMO Medical Tech Co., Ltd, Chengdu Kelong Chemical company, Shaoyuan Chemical Technology company, Bailingwei Technology company, etc.

Nitrogen atmosphere means that the reaction flask was connected with a nitrogen balloon with a volume of about 1L.

Hydrogen atmosphere means that the reaction flask was connected with a hydrogen balloon with a volume of about 1L.

Hydrogenation reaction was usually vacuumized, filled with hydrogen, and repeated for 3 times.

Unless otherwise stated in Examples, the reaction was carried out in nitrogen atmosphere.

Unless otherwise stated in Examples, the solution was an aqueous solution.

Unless otherwise stated in Examples, the reaction temperature was room temperature, and the most suitable reaction temperature for room temperature was 20°C-30°C.
DCM: dichloromethane;
EA: ethyl acetate;
HCl: hydrochloric acid;
THF: tetrahydrofuran;
DMF: N,N-dimethyl formamide;
PE: petroleum ether;
TLC: thin layer chromatography;
SFC: supercritical fluid chromatography;
NCS: N-chlorosuccinimide;
Pd(dppf)Cl₂: [1,1'-bis (diphenylphosphine) ferrocene] palladium dichloride;
DMSO: dimethyl sulfoxide;
DTT: dithiothreitol;
ATP: triphosadenine;
DNA: deoxynucleotide.

### EXAMPLES

The technical solution of the present application will be illustrated by the following Examples in detail, and the scope of the present application comprises, but is not limited to the examples.

### Example 1

### 4-methyl-5'-(methyl-d3)-2'-(prop-1-en-2-yl)-1',2',3',4'-tetrahydro-[1,1'-biphenyl]-2,6-diol (compound 1)

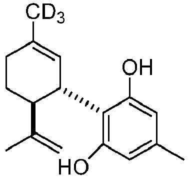

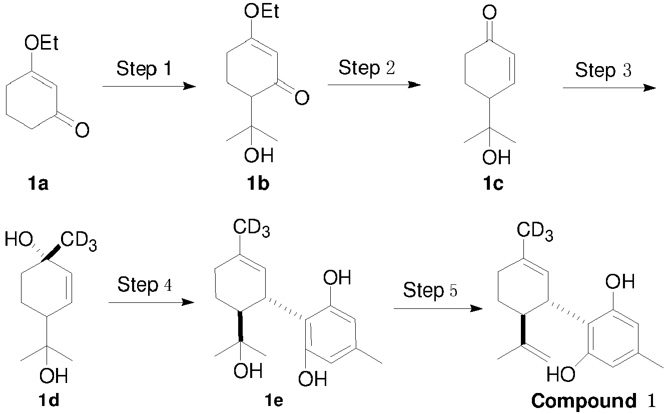

### Step 1:

### 3-ethoxy-6-(2-hydroxypropan-2-yl)cyclohex-2-en-1-one 1b

Under nitrogen gas atmosphere, 3-ethoxycyclohex-2-en-1-one 1a (10.0 g, 71.0 mmol) was added dropwise to lithium diisopropylamide (54 mL, 107.0 mmol, 2.0 N) in tetrahydrofuran (100 mL) at -70°C, and the mixture was stirred for 0.5 h after the addition was completed. Then acetone (9.2 g, 142.0 mmol) was added dropwise, and the mixture was stirred for 3 hours after the addition was completed. TLC test was used to monitor the reaction until the reaction was completed. Saturated solution of ammonium chloride (180 mL) was added dropwise to quench the reaction, and the thus obtained reaction solution was separated. The aqueous phase was extracted with ethyl acetate (150 mL×2). The organic phases were combined and washed with saturated brine (100 mL), dried over anhydrous sodium sulfate, and filtered. The filtrate was concentrated under reduced pressure to obtain titled compound 1b (yellow oil, 18.0 g, with a yield of 99.0%).

¹H NMR (400 MHz, DMSO) δ 5.29 (s, 1H), 4.93 (s, 1H), 3.98-3.87 (m, 2H), 2.46 (dd, J = 11.2, 5.0 Hz, 1H), 2.37 (dt, J = 17.4, 4.5 Hz, 1H), 2.21 (dd, J = 12.1, 4.7 Hz, 1H), 2.12-2.03 (m, 1H), 1.67 (ddd, J = 24.2, 12.3, 5.1 Hz, 1H), 1.27 (t, J = 7.0 Hz, 3H), 0.99 (s, 3H), 0.73 (s, 3H).

LC-MS m/z (ESI) = 199.2 [M+1].

### Step 2:

### 4-(2-hydroxypropan-2-yl)cyclohex-2-en-1-one 1c

Under nitrogen gas atmosphere, red aluminum (67 mL, 234.1 mmol, 3.5 N) was added dropwise to compound 1b (17.0 g, 78.0 mmol) in tetrahydrofuran (200 mL) at 0°C, and the mixture was slowly heated to room temperature and stirred for 2 hours. TLC test was used to monitor the reaction until the reaction was completed. The thus obtained mixture was cooled to 0°C, quenched by dropwise adding saturated solution of ammonium chloride (13 mL), and filtered by suction. The aqueous phase was extracted with ethyl acetate (200 mL×2). The organic phases were combined, washed with saturated brine (100 mL), dried over anhydrous sodium sulfate and filtered. The filtrate was concentrated under reduced pressure to obtain a crude product which was then dissolved in tetrahydrofuran (40 mL), then hydrochloric acid aqueous solution (40 mL, 2 N) was added dropwise thereto and the thus obtained mixture was stirred at room temperature for 1.5 hours after the addition was completed. TLC test was used to monitor the reaction until the reaction was completed. The reaction was quenched by dropwise adding saturated solution of sodium bicarbonate. The thus obtained mixture was concentrated under reduced pressure, the aqueous phase was extracted with ethyl acetate (100 mL×4), and the combined organic phases were washed with saturated brine (100 mL), dried over anhydrous sodium sulfate, and filtered. The filtrate was concentrated under reduced pressure and purified by silica gel column chromatography (eluent: ethyl acetate/petroleum ether (v/v)=1/3) to obtain titled compound 1c (colorless oil, 5.8 g, with a yield of 48.0%).

¹H NMR (400 MHz, DMSO) δ 7.19 (dt, J = 10.4, 1.9 Hz, 1H), 5.93 (dd, J = 10.4, 2.8 Hz, 1H), 4.58 (s, 1H), 2.44-2.38 (m, 1H), 2.38-2.29 (m, 2H), 2.08-1.96 (m, 1H), 1.62 (tdd, J = 12.9, 9.4, 5.0 Hz, 1H), 0.99 (s, 3H), 0.73 (s, 3H).

LC-MS m/z (ESI) = 155.4 [M+1].

### Step 3:

### 4-(2-hydroxypropan-2-yl)-1-(methyl-d3)cyclohex-2-en-1-ol 1d

Under nitrogen gas atmosphere, deuterated methyl magnesium iodide (34 mL, 33.7 mmol, 1.0 N) was added dropwise to anhydrous lithium chloride (1.4 g, 33.7 mmol) in tetrahydrofuran (20 mL) at 0°C, and the mixture was stirred for 0.5 hour after the addition was completed. Compound 1c (1.8 g, 11.2 mmol) in tetrahydrofuran solution (5 mL) was dropwise added to the mixture and further reacted under stirring for 0.5 hour. TLC test was used to monitor the reaction until the reaction was completed. Saturated solution of ammonium chloride (20 mL) was added dropwise to quench the reaction, and the aqueous phase was extracted with ethyl acetate (50 mL×3). The organic phases were combined and washed with saturated brine (50 mL), dried over anhydrous sodium sulfate, and filtered. The filtrate was concentrated under reduced pressure to obtain the crude product which was purified by silica-gel column chromatography (eluent: ethyl acetate/petroleum ether (v/v)=2/1) to obtain titled compound 1d (white solid, 1.2 g, with a yield of 60.0%).

¹H NMR (400 MHz, DMSO) δ 5.61 (d, J = 10.4 Hz, 1H), 5.52 (d, J = 10.6 Hz, 1H), 4.38 (s, 1H), 4.17 (s, 1H), 2.00 (ddd, J = 10.8, 5.1, 2.4 Hz, 1H), 1.73-1.65 (m, 2H), 1.57-1.48 (m, 1H), 1.21 (dd, J = 10.1, 6.8 Hz, 1H), 0.99 (s, 3H), 0.73 (s, 3H).

LC-MS m/z (ESI) = 174.6 [M+1].

### Step 4:

### 2'-(2-hydroxypropan-2-yl)-4-methyl-5'-(methyl-d3)-1',2',3',4'-tetrahydro-[1,1'-biphenyl]-2,6-diol 1e

4-(2-hydroxypropyl-2-yl)-1-(methyl-d3) cyclohex-2-ene-1-ol 1d (500 mg, 2.9 mmol), 5-methylbenzene-1,3-diol (1080mg, 8.7mmol) and 4A molecular sieve (1.5g) were dissolved in 10 mL of dichloromethane, the atmosphere in the flask was replaced by nitrogen gas and the thus obtained mixture was stirred for 10 minutes at room temperature. Then, L-camphor sulfonic acid (67.34 mg, 0.29 mmol) was added thereto. The thus obtained mixture was stirred for 1 hour at room temperature, then quenched by adding 20mL of saturated aqueous solution of sodium bicarbonate, and the thus obtained mixture was extracted with ethyl acetate (10mL×4). Organic phases were combined, dried over anhydrous sodium sulfate, and filtered. The filtrate was concentrated to obtain 700mg crude product which was purified by column chromatography to obtain 2'-(2-hydroxypropan-2-yl)-4-methyl-5'-(methyl-d3)-1',2',3',4'-tetrahydro-[1,1'-biphenyl]-2,6-diol 1e (white solid, 375mg, with a yield of 46%).

¹H NMR (400 MHz, DMSO-d6) δ 9.03 (s, 1H), 8.79 (s, 1H), 6.09 (d, 1H), 5.95 (d, 1H), 4.92 (s, 1H), 3.90 (d, 1H), 2.14 (d, 5H), 1.37-1.21 (m, 2H), 0.99 (s, 3H), 0.91-0.79 (m, 2H), 0.73 (s, 3H).

### Step 5:

### 4-methyl-5'-(methyl-d3)-2'-(prop-1-en-2-yl)-1',2',3',4'-tetrahydro-[1,1'-biphenyl]-2,6-diol (compound 1)

2'-(2-hydroxypropan-2-yl)-4-methyl-5'-(methyl-d3)-1',2',3',4'-tetrahydro-[1,1'-biphenyl]-2,6-diol 1e (350 mg, 1.25 mmol) was dissolved in 6 mL of tetrahydrofuran, the atmosphere in the flask was replaced by nitrogen gas and the thus obtained system was cooled to 0°C. Then, Burgess reagent (595 mg, 2.5 mmol) was added into the system and the thus obtained mixture was warmed to room temperature. The reaction was performed for 20 minutes, followed by quenching the reaction by adding 20mL of saturated aqueous solution of sodium bicarbonate, and the thus obtained mixture was extracted with ethyl acetate (10mL×4). Organic phases were combined, dried over anhydrous sodium sulfate, filtered and concentrated to obtain 500mg of crude product, so as to obtain 4-methyl-5'-(methyl-d3)-2'-(prop-1-en-2-yl)-1',2',3',4'-tetrahydro-[1,1'-biphenyl]-2,6-diol (compound 1) (purple solid, 30mg, with a yield of 9.2%).

¹H NMR (400 MHz, Chloroform-d) δ 6.28-6.15 (m, 2H), 5.54 (d, 1H), 4.65 (p, 1H), 4.53-4.37 (m, 1H), 3.61-3.46 (m, 1H), 2.45 (ddd, 1H), 2.22 (ddt, 1H), 2.14 (s, 3H), 2.10 (q, 1H), 1.86-1.69 (m, 2H), 1.56 (t, 3H).

### Example 2

### 4-ethyl-5'-(methyl-d3)-2'-(prop-1-en-2-yl)-1',2',3',4'-tetrahydro-[1,1'-biphenyl]-2,6-diol (compound 2)

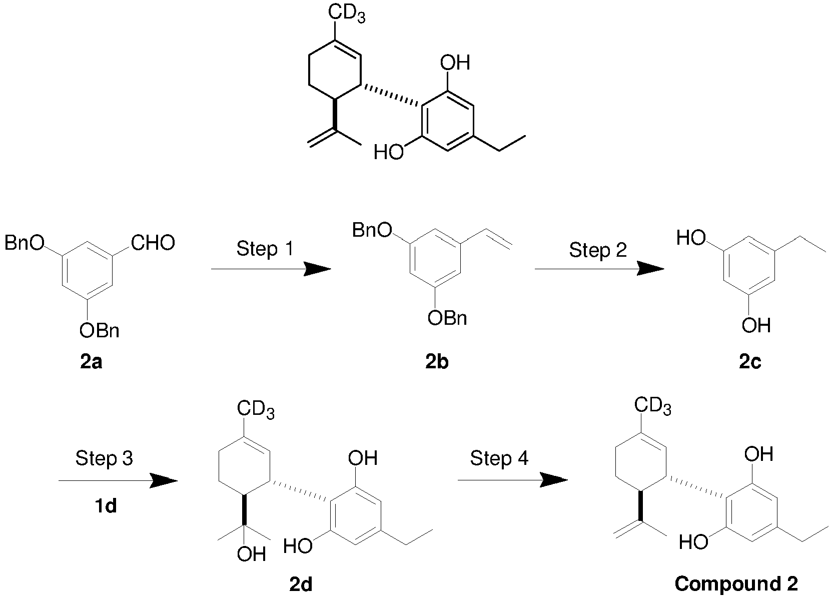

### Step 1:

### (((5-Vinyl-1,3-phenylene)bis(oxy))bis(methylene))dibenzene 2b

T-BuOK(1.5 g, 13.0 mmol) was added to methyltriphenylphosphonium bromide (5.0 g, 13.0 mmol) in THF at 0°C, followed by stirring for 0.5 hour at 0°C. Then compound 2a (3.0 g, 10.8 mmol) was added thereto, followed by further reacting under stirring at room temperature until the reaction was completed. Then, the reaction mixture was extracted with EA, dried over anhydrous Na₂SO₄, and concentrated under vacuum to obtain a crude product which was purified by column chromatography to obtain compound 2b (white solid, 3.0 g, 88.8%).

¹H NMR (400 MHz, DMSO): δ (ppm) 7.58-7.30 (m, 10H), 6.68 (d, 2H), 6.67-6.60 (m, 1H), 6.56 (t, 1H), 5.72 (d, 1H), 5.26 (d, 1H), 5.05 (s, 4H).

LC-MS m/z (ESI) = 317.20 [M+1].

### Step 2:

### 5-ethylbenzene-1,3-diol 2c

Compound 2b (3.0 g, 9.5 mmol) was dissolved in the mixed solution of EA and methanol (v1/v2=5/1), then 10% Pd/C (600.0 mg) was added and the resultant mixture was stirred overnight under H₂ condition. After the reaction was completed, the reaction mixture was filtered and the filtrate was extracted with EA. The organic phases were dried over anhydrous Na₂SO₄, concentrated under vacuum and purified by column chromatography to obtain compound 2c (white solid, 1.2 g, 91.7%).

¹H NMR (400 MHz, DMSO): δ (ppm) 6.27 (d, 2H), 6.20 (t, 1H), 4.14 (q, 1H), 2.51 (q, 2H), 1.16 (t, 3H).

LC-MS m/z (ESI) = 139.10 [M+1].

### Step 3:

### 4-ethyl-2'-(2-hydroxypropan-2-yl)-5'-(methyl-d3)-1',2',3',4'-tetrahydro-[1,1'-biphenyl]-2,6-diol 2d

Compound 2c (1.2 g, 8.7 mmol) and L-camphor sulfonic acid (69.7 mg, 0.3 mmol) were dissolved in anhydrous DCM and stirred for 10 minutes, then compound 1d (502.1 mg, 2.9 mmol) was added to the above solution. After the gas in the reaction container was replaced for three times, the solution was stirred at room temperature for about 1 hour. The reaction solution was adjusted to pH 7~8 with saturated solution of NaHCO₃, extract with dichloromethane, dried over anhydrous Na₂SO₄, and concentrated under vacuum to obtain a crude product which was purified by column chromatography to obtain compound 2d (white solid, 200.0 mg, 23.5%).

¹H NMR (400 MHz, DMSO): δ (ppm) 6.50 (s, 1H), 6.39-6.25 (m, 2H), 5.70-5.62 (m, 1H), 3.90-3.79 (m, 1H), 2.50 (q, 2H), 2.18-1.86 (m, 4H), 1.75-1.64 (m, 1H), 1.23 (s, 6H), 1.18 (t, 3H).

LC-MS m/z (ESI) = 316.20 [M+23].

### Step 4:

### 4-ethyl-5'-(methyl-d3)-2'-(prop-1-en-2-yl)-1',2',3',4'-tetrahydro-[1,1'-biphenyl]-2,6-diol (compound 2)

4-ethyl-5'-(methyl-d3)-2'-(prop-1-en-2-yl)-1',2',3',4'-tetrahydro-[1,1'-biphenyl]-2,6-diol

Compound 2d (200.0mg, 0.7 mmol) was weighed and taken into the reaction flask, and atmosphere in the reaction flask was replaced for three times. 5 mL of THF was added to the resultant mixture at room temperature, followed by stirring until the solid was completely dissolved. Burgess reagent (333.6 mg, 1.4 mmol) was slowly added in an ice bath, and then the reaction flask was moved to room temperature and stirred for about 20 minutes after the addition was completed. TLC test was used to monitor the reaction until compound 2d was consumed completely. The reaction mixture was adjusted to pH 7~8 by adding saturated solution of NaHCO₃, and extracted with ethyl acetate. The resultant organic phases were washed with saturated solution of NaCl, dried over anhydrous Na₂SO₄, concentrated and purified by column chromatography to obtain crude product which was purified by preparative chromatographic column to obtain target compound 2 (purple solid, 16.0 mg, 35.5%).

¹H NMR (400 MHz, DMSO): δ (ppm) 8.67 (s, 2H), 6.04 (s, 2H), 5.08 (s, 1H), 4.50 (d, 1H), 4.41 (dd, 1H), 3.91-3.77 (m, 1H), 3.09-2.98 (m, 1H), 2.34 (q, 2H), 2.21-2.02 (m, 1H), 1.98-1.86 (m, 1H), 1.73-1.60 (m, 2H), 1.59 (s, 3H), 1.08 (t, 3H).

LC-MS m/z (ESI) = 270.20 [M+1].

### Example 3

### 4-ethyl-5'-(methyl-d3)-2'-(prop-1-en-2-yl)-1',2',3',4'-tetrahydro-[1,1'-biphenyl]-2,6-diol (compound 3)

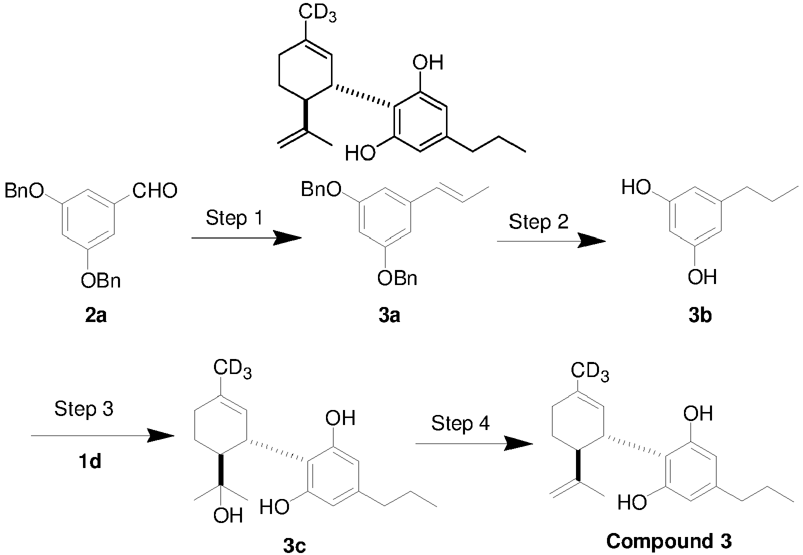

### Step 1:

### (E)-(((5-(prop-1-en-1-yl)-1,3-phenylene)bis(oxy))bis(methylene))dibenzene 3a

T-BuOK (1.4 g, 12.5 mmol) was added to methyltriphenylphosphonium bromide (4.5 g, 12.1 mmol) in THF at 0°C, followed by stirring for 0.5 hour at 0°C. Then compound 2a (3.2 g, 10.1 mmol) was added thereto, followed by further reacting under stirring at room temperature until the reaction was completed. Then, the reaction mixture was extracted with EA, dried over anhydrous Na₂SO₄, and concentrated under vacuum to obtain a crude product which was purified by column chromatography to obtain compound 3a (white solid, 3.0 g, 90.3%).

¹H NMR (400 MHz, DMSO): δ (ppm) 7.50-7.30 (m, 10H), 6.61 (d, 1H), 6.56 (d, 2H), 6.54 (t, 1H), 6.37 (dd, 1H), 5.78 (dd, 1H), 5.05 (s, 4H), 1.85 (dd, 3H).

LC-MS m/z (ESI) = 331.20 [M+1].

### Step 2:

### 5-propylbenzene-1,3-diol 3b

Compound 3a (3.0 g, 9.1 mmol) was dissolved in the mixed solution of EA and methanol (v1/v2=5/1), then 10% Pd/C (300.0 mg) was added and the resultant mixture was stirred overnight under H₂ condition. After the reaction was completed, the reaction mixture was filtered and the filtrate was extracted with EA. The resultant organic phases were concentrated under vacuum and purified by column chromatography to obtain the corresponding target compound 3b (white solid, 1.3 g, 96.3%).

¹H NMR (400 MHz, DMSO): δ (ppm) (d, 2H), 6.18 (t, 1H), 2.47 (dd, 2H), 1.59 (dt, 2H), 0.93 (t, 3H).

LC-MS m/z (ESI) = 153.10 [M+1].

### Step 3:

### 2'-(2-hydroxypropan-2-yl)-5'-(methyl-d3)-4-propyl-1',2',3',4'-tetrahydro-[1,1'-biphenyl]-2,6-diol 3c

Compound 3b (1.3 g, 8.7 mmol) and L-camphor sulfonic acid (67.1 mg, 0.3 mmol) were dissolved in anhydrous DCM and stirred for 10 minutes, then compound 1d (502.1 mg, 2.9 mmol) was added to the above solution, following by stirring at room temperature for about 1 hour after the gas in the reaction container was replaced for three times. After the reaction was completed, the reaction solution was adjusted to pH 7~8 with saturated solution of NaHCO₃, extracted with DCM, dried over anhydrous Na₂SO₄, and concentrated under vacuum to obtain a crude product which was purified by column chromatography to obtain target compound 3c (white solid, 300.0 mg, 11.3%).

¹H NMR (400 MHz, DMSO): δ (ppm) 6.50 (s, 1H), 6.29 (d, 2H), 5.72-5.65 (m, 1H), 3.89-3.81 (m, 1H), 2.44 (t, 2H), 2.18-1.87 (m, 4H), 1.77-1.67 (m, 1H), 1.66-1.53 (m, 2H), 1.24 (s, 6H), 0.92 (t, 3H).

LC-MS m/z (ESI)= 330.20[M+23].

### Step 4:

### 5'-(methyl-d3)-2'-(prop-1-en-2-yl)-4-propyl-1',2',3',4'-tetrahydro-[1,1'-biphenyl]-2,6-diol (compound 3)

Compound 3c (350.0mg, 1.2mmol) was weighed and taken into the reaction flask, and the atmosphere therein was replaced with N₂ for three time. 5 mL of THF was added to the resultant mixture at room temperature, followed by stirring until the solid was completely dissolved. Burgess reagent (571.9 mg, 2.4 mmol) was slowly added in an ice bath, and then the reaction flask was moved to room temperature and stirred for about 20 minutes after the addition was completed. TLC test was used to monitor the reaction until compound 3c was consumed completely. The reaction solution was adjusted to pH 7~8 with saturated solution of NaHCO₃ and extracted with ethyl acetate. The organic phases were washed with saturated solution of NaCl, dried over anhydrous Na₂SO₄, concentrated and purified with column chromatography to obtain a crude product which was purified by preparative chromatographic column to obtain target compound 3 (purple solid, 20.0 mg, 6.1%).

¹H NMR (400 MHz, DMSO): δ (ppm) 8.66 (s, 2H), 6.01 (s, 2H), 5.08 (s, 1H), 4.49 (d, 1H), 4.40 (dd, 1H), 3.87-3.75 (m, 1H), 3.09-2.96 (m, 1H), 2.28 (t, 2H), 2.14 (s, 1H), 1.96-1.87 (m, 1H), 1.73-1.60 (m, 2H), 1.58 (s, 3H), 1.55-1.41 (m, 2H), 0.86 (t, 3H).

LC-MS m/z (ESI) = 290.20 [M+23].

### Example 4

### 4-butyl-5'-(methyl-d3)-2'-(prop-1-en-2-yl)-1',2',3',4'-tetrahydro-[1,1'-biphenyl]-2,6-diol (compound 4)

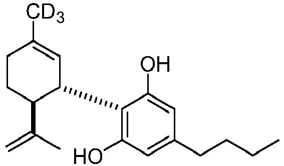

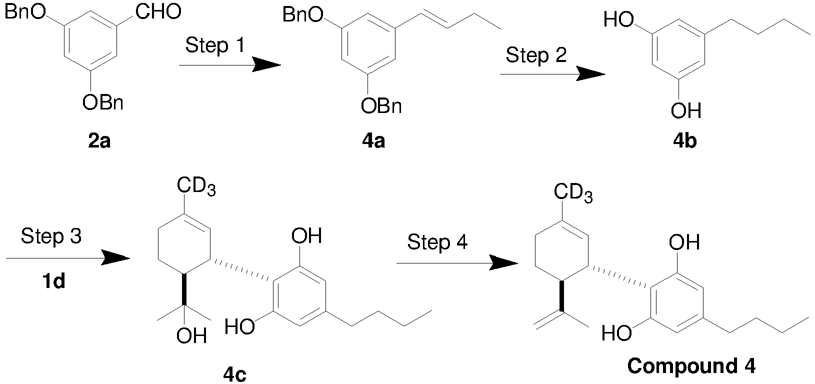

### Step 1:

### (E)-(((5-(but-1-en-1-yl)-1,3-phenylene)bis(oxy))bis(methylene))dibenzene 4a

T-BuOK (1.5 g, 13.0 mmol) was added to methyl triphenyl phosphonium bromide (5.0 g, 13.0 mmol) in THF at 0°C, followed by stirring for 0.5 hour at 0°C. Then compound 2a (3.4 g, 10.8 mmol) was added thereto, followed by further reacting under stirring at room temperature until the reaction was completed. Then, the reaction mixture was extracted with EA, dried over anhydrous Na₂SO₄, and concentrated under vacuum to obtain a crude product which was purified by column chromatography to obtain compound 4a (white solid, 3.6 g, 96.7%).

¹H NMR (400 MHz, DMSO): δ (ppm) 7.47-7.29 (m, 10H), 6.52 (s, 2H), 6.34-6.23 (m, 1H), 5.67-5.58 (m, 1H), 5.04 (s, 4H), 2.34-2.18 (m, 2H), 1.02 (t, 3H).

LC-MS m/z (ESI) = 345.20 [M+1].

### Step 2:

### 5-butylbenzene-1,3-diol 4b

Compound 4a (3.6 g, 10.4 mmol) was dissolved in the mixed solution of EA and methanol (v1/v2=5/1), then 10% Pd/C (360.0 mg) was added and the resultant mixture was stirred overnight under H₂ condition. After the reaction was completed, the reaction mixture was filtered and the filtrate was extracted with EA. The resultant organic phases were concentrated under vacuum and purified by column chromatography to obtain the corresponding target compound 4b (white solid, 1.4 g, 80.6%).

¹H NMR (400 MHz, DMSO): δ (ppm) (d, 2H), 6.18 (t, 1H), 2.47 (dd, 2H), 1.59 (dt, 2H), 0.93 (t, 3H).

LC-MS m/z (ESI) = 167.1 [M+1].

### Step 3:

### 4-butyl-2'-(2-hydroxypropan-2-yl)-5'-(methyl-d3)-1',2',3',4'-tetrahydro-[1,1'-bi-phenyl]-2,6-diol 4c

Compound 4b (1.4 g, 4.3 mmol) and L-camphor sulfonic acid (67.1 mg, 0.3 mmol) were dissolved in anhydrous DCM solution and stirred for 10 minutes, then compound 1d (502.1 mg, 2.9 mmol) was added to the above solution, following by stirring at room temperature for about 1 hour after the gas in the reaction container was replaced for three times. After the reaction was completed, the reaction solution was adjusted to pH 7~8 with saturated solution of NaHCO₃, extracted with DCM, dried over anhydrous Na₂SO₄, and concentrated under vacuum to obtain a crude product which was purified by column chromatography to obtain target compound 4c (white solid, 250 mg, 9.2%).

¹H NMR (400 MHz, Chloroform-d): δ (ppm) 6.48 (s, 1H), 6.36-6.24 (m, 2H), 5.73-5.64 (m, 1H), 3.88-3.80 (m, 1H), 2.51-2.41 (m, 2H), 2.20-1.86 (m, 4H), 1.76-1.65 (m, 1H), 1.61-1.49 (m, 2H), 1.37-1.30 (m, 2H), 1.23 (s, 6H), 0.90 (t, 3H).

LC-MS m/z (ESI) = 343.20[M+23].

### Step 4:

### 4-butyl-5'-(methyl-d3)-2'-(prop-1-en-2-yl)-1',2',3',4'-tetrahydro-[1,1'-biphenyl]-2,6-diol (compound 4)

Compound 4c (250.0 mg, 0.8 mmol) was weighed and taken into the reaction flask, and the atmosphere in the reaction flask was replaced for three times. 5 mL of THF was added to the resultant mixture at room temperature, followed by stirring until the solid was completely dissolved. Burgess reagent (381.3 mg, 1.6 mmol) was slowly added in an ice bath, and then the reaction flask was moved to room temperature and stirred for about 20 minutes. TLC test was used to monitor the reaction until compound 4c was consumed completely. The reaction mixture was adjusted to pH 7~8 by adding NaHCO₃, and extracted with EA. The organic phases were washed with saturated solution of NaCl, dried over anhydrous Na₂SO₄, concentrated and purified by column chromatography to obtain a crude product which was purified by preparative chromatographic column to obtain target compound 4 (purple solid, 25 mg, 10.6%).

¹H NMR (400 MHz, DMSO): δ (ppm) 8.66 (s, 2H), 6.01 (s, 2H), 5.07 (s, 1H), 4.49 (d, 1H), 4.40 (dd, 1H), 3.90-3.75 (m, 1H), 3.07-2.96 (m, 1H), 2.30 (t, 2H), 2.19-2.02 (m, 1H), 1.99-1.84 (m, 1H), 1.73-1.60 (m, 2H), 1.58 (s, 3H), 1.51-1.39 (m, 2H), 1.36-1.20 (m, 2H), 0.87 (t, 3H).

LC-MS m/z (ESI) = 304.20 [M+23].

### Example 5

### Methyl-2,6-dihydroxy-5'-(methyl-d3)-4-pentyl-2'-(prop-1-en-2-yl)-1',2',3',4'-tetrahydro-[1,1'-biphenyl]-3-carboxylate (compound 5)

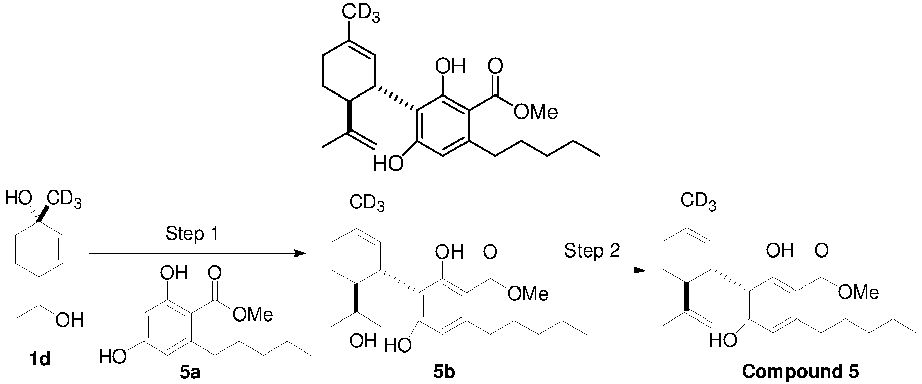

### Step 1:

### Methyl-2,6-dihydroxy-2'-(2-hydroxypropan-2-yl)-5'-(methyl-d3)-4-pentyl-1',2',3',4'-tetrahydro-[1,1'-biphenyl]-3-carboxylate 5b

Compound 1d (150 mg, 0.86 mmol), compound 5a (619 mg, 2.6 mmol) and 4A molecular sieves (450mg) were added into the flask, and L-camphorsulfonic acid (19 mg, 0.086 mmol), was added thereto under nitrogen gas atmosphere. The resultant mixture was stirred at room temperature and the reaction was completed in 1.5 hours. The resultant product was purified by column chromatography (PE:EA=15:1) to obtain target product 5b (yellow oil, 207 mg, with a yield of 61.4%).

¹H NMR (400 MHz, DMSO-d6) δ 11.51 (s, 1H), 9.87 (s, 1H), 6.21 (s, 1H), 4.87 (d, 1H), 3.83 (s, 3H), 3.75 (d, 1H), 2.71-2.63 (m, 2H), 2.38 (td, 1H), 2.16-1.98 (m, 2H), 1.92-1.81 (m, 1H), 1.45 (q, 2H), 1.28 (ddd, 10.5, 6H), 0.97 (s, 3H), 0.86 (t, 3H), 0.79 (s, 3H).

### Step 2:

### Methyl-2,6-dihydroxy-5'-(methyl-d3)-4-pentyl-2'-(prop-1-en-2-yl)-1',2',3',4'-tetrahydro-[1,1'-biphenyl]-3-carboxylate (compound 5)

Compound 5b (207 mg, 0.52 mmol) and tetrahydrofuran (3 mL) were added into the flask, and Burgess reagent (250 mg, 1.01 mmol) was added thereto at 0 °C under nitrogen gas atmosphere. The reaction was completed after 20 minutes. Then, the reaction was quenched with 5 mL of sodium bicarbonate aqueous solution, and column chromatography separation was performed to obtain target product compound 5 (white solid product, 66 mg, with a yield of 33.5%).

¹H NMR (400 MHz, DMSO-d6) δ 11.54 (s, 1H), 9.90 (s, 1H), 6.20 (s, 1H), 5.09 (d, 1H), 4.52-4.36 (m, 2H), 3.96-3.86 (m, 1H), 3.83 (s, 4H), 3.02 (td, 11.0, 1H), 2.73-2.62 (m, 2H), 2.18-2.07 (m, 1H), 2.00-1.91 (m, 1H), 1.75-1.60 (m, 2H), 1.58 (s, 3H), 1.44 (t, 2H), 1.32-1.26 (m, 4H), 0.90-0.84 (m, 3H).

### Example 6

### Ethyl-2,6-dihydroxy-5'-(methyl-d3)-4-pentyl-2'-(prop-1-en-2-yl)-1',2',3',4'-tetrahydro-[1,1'-biphenyl]-3-carboxylate (compound 6)

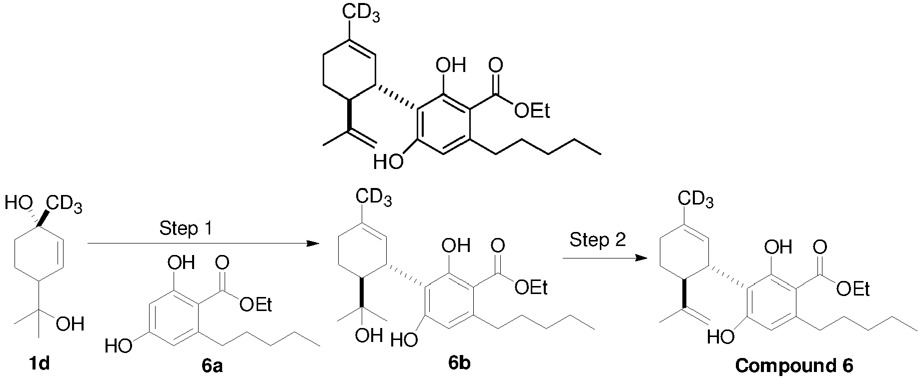

### Step 1:

### Ethyl-2,6-dihydroxy-2'-(2-hydroxypropan-2-yl)-5'-(methyl-d3)-4-pentyl-1',2',3',4'-tetrahydro-[1,1'-biphenyl]-3-carboxylate 6b

Compound 1d (150 mg, 0.86 mmol), compound 6a (655 mg, 2.6 mmol) and 4A molecular sieves (450mg) were added into the flask, and L-camphorsulfonic acid (19 mg, 0.086 mmol), was added thereto under nitrogen gas atmosphere. The resultant mixture was stirred at room temperature, and the reaction was completed in 1.5 hours. Column chromatography separation was performed (PE:EA=15:1) to obtain target product 6b (yellow oil, 200 mg, with a yield of 57.1%).

¹H NMR (400 MHz, DMSO-d6) δ 11.77 (d, 1H), 9.89 (s, 1H), 6.21 (s, 1H), 4.86 (d, 1H), 4.32 (q, 2H), 3.80 (s, 2H), 2.71 (s, 2H), 2.39 (td, 1H), 2.16-1.99 (m, 2H), 1.92-1.80 (m, 1H), 1.49-1.42 (m, 2H), 1.36-1.29 (m, 7H), 0.97 (s, 4H), 0.88-0.84 (m, 3H), 0.79 (s, 3H).

### Step 2:

### Ehyl-2,6-dihydroxy-5'-(methyl-d3)-4-pentyl-2'-(prop-1-en-2-yl)-1',2',3',4'-tetrahydro-[1,1'-biphenyl]-3-carboxylate (compound 6)

Compound 6b (200 mg, 0.51 mmol) and tetrahydrofuran (3 mL) were added into the flask, and Burgess reagent (254 mg, 1.02 mmol) was added thereto at 0°C under nitrogen gas atmosphere. The reaction was completed after 20 minutes, then quenched with 5 mL of sodium bicarbonate aqueous solution. Column chromatography separation was performed to obtain target product compound 6 (white solid product, 66 mg, with a yield of 33.5%).

¹H NMR (400 MHz, DMSO-d6) δ 11.78 (s, 1H), 9.91 (s, 1H), 6.19 (s, 1H), 5.08 (d, 1H), 4.47-4.42 (m, 2H), 4.33 (t, 2H), 3.90 (d, 1H), 3.02 (td, 10.9, 1H), 2.69 (d, 2H), 2.15-2.09 (m, 1H), 1.98-1.91 (m, 1H), 1.71-1.61 (m, 2H), 1.58 (s, 3H), 1.48-1.43 (m, 2H), 1.31 (d, 3H), 1.29-1.24 (m, 4H), 0.87 (q, 3H).

### Example 7

### (1R,2'R)-5'-(methyl-d3)-4-pentyl-2'-(prop-1-en-2-yl-d5)-1',2',3',4'-tetrahydro-[1,1'-biphenyl]-2,6-diol (compound 7)

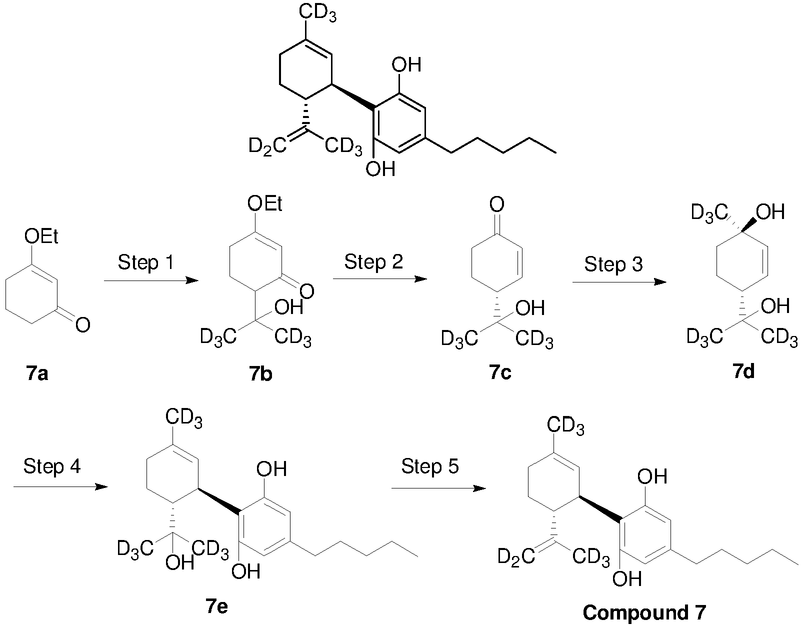

### Step 1:

### 3-ethoxy-6-(2-hydroxypropan-2-yl-1,1,1,3,3,3-d6)cyclohex-2-en-1-one 7b

Under nitrogen gas atmosphere, 3-ethoxycyclohex-2-en-1-one 7a (10.0 g, 71.0 mmol) was added dropwise to lithium diisopropylamide (54 mL, 107.0 mmol, 2.0 N) in tetrahydrofuran (100 mL) at -70°C, and the mixture was stirred for 0.5 hour after the addition was completed. Then deuterated acetone (9.2 g, 142.0 mmol) was added dropwise, and the mixture was stirred for 3 hours after the addition was completed. TLC test was used to monitor the reaction until the reaction was completed. Saturated solution of ammonium chloride (180 mL) was added dropwise to quench the reaction, and the reaction solution was separated. The aqueous phase was extracted with ethyl acetate (150 mL×2). The organic phases were combined and washed with saturated brine (100 mL), dried over anhydrous sodium sulfate, and filtered. The filtrate was concentrated under reduced pressure to obtain titled compound 7b (yellow oil, 18.0 g, with a yield of 99.0%).

¹H NMR (400 MHz, DMSO). δ 5.29 (s, 1H), 4.93 (s, 1H), 3.98-3.87 (m, 2H), 2.46 (dd, J = 11.2, 5.0 Hz, 1H), 2.37 (dt, J = 17.4, 4.5 Hz, 1H), 2.21 (dd, J = 12.1, 4.7 Hz, 1H), 2.12-2.03 (m, 1H), 1.67 (ddd, J = 24.2, 12.3, 5.1 Hz, 1H), 1.27 (t, J = 7.0 Hz, 3H).

LC-MS m/z (ESI) = 205.1 [M+1].

### Step 2:

### (R)-4-(2-hydroxypropan-2-yl-1,1,1,3,3,3-d6)cyclohex-2-en-1-one 7c

Under nitrogen gas atmosphere, red aluminum (67 mL, 234.1 mmol, 3.5 N) was added dropwise to compound 7b (17.0 g, 78.0 mmol) in tetrahydrofuran (200 mL) at 0°C, and the mixture was slowly warmed to room temperature and stirred for 2 hours after the addition was completed. TLC test was used to monitor the reaction until the reaction was completed. The thus obtained mixture was cooled to 0°C, quenched by dropwise adding saturated solution of ammonium chloride (13 mL), and suction filtered. The aqueous phase was extracted with ethyl acetate (200 mL×2). The organic phases were combined, washed with saturated brine (100 mL), dried over anhydrous sodium sulfate and filtered. The filtrate was concentrated under reduced pressure to obtain a crude product which was dissolved in tetrahydrofuran (40 mL), hydrochloric acid aqueous solution (40 mL, 2 N) was added dropwise thereto and the thus obtained mixture was stirred at room temperature for 1.5 hours after the addition was completed. TLC test was used to monitor the reaction until the reaction was completed. The reaction was quenched by dropwise adding saturated solution of sodium bicarbonate. The thus obtained mixture was concentrated under reduced pressure, the aqueous phase was extracted with ethyl acetate (100 mL×4), and the combined organic phases were washed with saturated brine (100 mL), dried over anhydrous sodium sulfate, and filtered. The filtrate was concentrated under reduced pressure and purified by silica gel column chromatography (eluent: ethyl acetate/petroleum ether (v/v)=1/3). The obtained racemate was separated and purified by preparative liquid chromatography (AD column, n-hexane: isopropanol =95:5, 214 nm, 25°C, retention time: 8.611 minutes) to obtain titled compound 7c (colorless oil, 5.8 g, with a yield of 48.0%).

¹H NMR (400 MHz, DMSO) δ 7.19 (dt, J = 10.4, 1.9 Hz, 1H), 5.93 (dd, J = 10.4, 2.8 Hz, 1H), 4.58 (s, 1H), 2.44-2.38 (m, 1H), 2.38-2.29 (m, 2H), 2.08-1.96 (m, 1H), 1.62 (tdd, J = 12.9, 9.4, 5.0 Hz, 1H).

LC-MS m/z (ESI) = 161.1 [M+1].

### Step 3:

### (1R,4R)-4-(2-hydroxypropan-2-yl-1,1,1,3,3,3-d6)-1-(methyl-d3)cyclohex-2-en-1-ol 7d

Under nitrogen gas atmosphere, deuterated methyl magnesium iodide (34 mL, 33.7 mmol, 1.0 N) was added dropwise to anhydrous lithium chloride (1.4 g, 33.7 mmol) in tetrahydrofuran (20 mL) at 0°C, and the mixture was stirred for 0.5 hour after the addition was completed. Compound 7c (1.8 g, 11.2 mmol) in tetrahydrofuran (5 mL) was dropwise added to the mixture and further reacted under stirring for 0.5 hour after the addition was completed. TLC test was used to monitor the reaction until the reaction was completed. Saturated solution of ammonium chloride (20 mL) was added dropwise to quench the reaction, and the aqueous phase was extracted with ethyl acetate (50 mL×3). The organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=2/1)to obtain titled compound 7d (white solid, 1.2 g, with a yield of 60.0%).

¹H NMR (400 MHz, DMSO) δ 5.61 (d, J = 10.4 Hz, 1H), 5.52 (d, J = 10.6 Hz, 1H), 4.38 (s, 1H), 4.17 (s, 1H), 2.00 (ddd, J = 10.8, 5.1, 2.4 Hz, 1H), 1.73-1.65 (m, 2H), 1.57-1.48 (m, 1H), 1.21 (dd, J = 10.1, 6.8 Hz, 1H).

### Step 4:

### (1'R,2'R)-2'-(2-hydroxypropan-2-yl-1,1,1,3,3,3-d6)-5-'(methyl-d3)-4-pentyl-1',2',3',4'-tetrahydro-[1,1'-biphenyl]-2,6-diol 7e

Under nitrogen gas atmosphere, L-(-) camphorsulfonic acid (65 mg, 0.28 mmol) was added dropwise to compound 7d (500 mg, 2.79 mmol), 3,5-dihydroxypentylbenzene (750 mg, 4.18 mmol) and 4A molecular sieve (1.5 g) in dichloromethane (10 mL), and after the addition was completed, the resultant mixture was reacted under stirring for 1.5 hours at room temperature. TLC test was used to monitor the reaction until the reaction was completed. Saturated solution of sodium bicarbonate (10 mL) was added dropwise to quench the reaction, and the aqueous phase was extracted with dichloromethane (30 mL×3). The organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/10) to obtain titled compound 7e (white solid, 380 mg, with a yield of 39.0%).

¹H NMR (400 MHz, DMSO) δ 8.73 (d, J = 110.7 Hz, 2H), 6.03 (s, 2H), 4.88 (s, 1H), 3.65 (s, 2H), 2.38-2.28 (m, 3H), 2.09 (d, J = 12.2 Hz, 1H), 2.03 -1.95 (m, 1H), 1.83 (d, J = 16.8 Hz, 1H), 1.51-1.43 (m, 2H), 1.30-1.21 (m, 5H), 0.85(t, J = 7.0 Hz, 3H).

LC-MS m/z (ESI) = 342.3 [M+1].

### Step 5:

### (1'R,2'R)-5'-(methyl-d3)-4-pentyl-2'-(prop-1-en-2-yl-d5)-1',2',3',4'-tetrahydro-[1,1'-biphenyl]-2,6-diol (compound 7)

Under nitrogen gas atmosphere, Burgess reagent (390 mg, 1.66 mmol) was added into compound 7e (380 mg, 1.10 mmol) in tetrahydrofuran (6 mL) at 0°C and the resultant mixture was warmed to room temperature and reacted under stirring for 20 minutes. TLC test was used to monitor the reaction until the reaction was completed. Saturated solution of sodium bicarbonate (10 mL) was added dropwise to quench the reaction, and the aqueous phase was extracted with ethyl acetate (30 mL×3). The organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/30) to obtain titled compound 7 (white solid, 44 mg; HPLC: 98%; with a yield of 12.0%).

¹H NMR (400 MHz, DMSO) δ 8.64 (s, 2H), 6.01 (s, 2H), 5.08 (s, 1H), 3.82 (dd, J = 8.8, 1.8 Hz, 1H), 3.07-2.97 (m, 1H), 2.32-2.27 (m, 2H), 2.14-2.04 (m, 1H), 1.91 (d, J = 16.8 Hz, 1H), 1.72-1.57 (m, 2H), 1.47 (dt, J = 14.8, 7.4 Hz, 2H),1.30-1.22 (m, 4H), 0.86 (t, J = 7.0 Hz, 3H).

LC-MS m/z (ESI)= 323.3 [M+1].

### Example 8

### (1'S,2'S)-5'-(methyl-d3)-4-pentyl-2'-(prop-1-en-2-yl-d5)-1',2',3',4'-tetrahydro-[1,1'-biphenyl]-2,6-diol (compound 8)

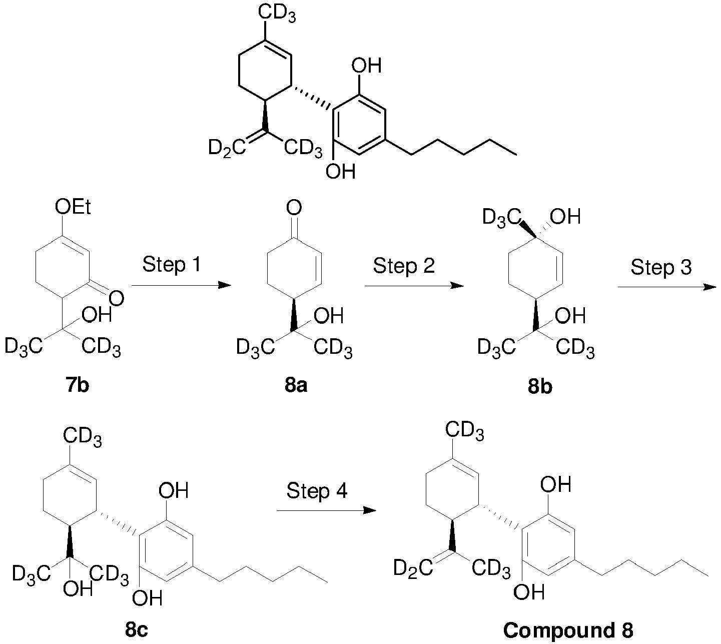

### Step 1:

### (S)-4-(2-hydroxypropan-2-yl-1,1,1,3,3,3-d6)cyclohex-2-en-1-one 8a

Under nitrogen gas atmosphere, red aluminum (67 mL, 234.1 mmol, 3.5 N) was added dropwise to compound 7b (17.0 g, 78.0 mmol) in tetrahydrofuran (200 mL) at 0°C, and the mixture was slowly warmed to room temperature and stirred for 2 hours after the addition was completed. TLC test was used to monitor the reaction until the reaction was completed. The thus obtained mixture was cooled to 0°C, quenched by dropwise adding saturated solution of ammonium chloride (13 mL), and suction filtered. The aqueous phase was extracted with ethyl acetate (200 mL×2). The organic phases were combined, washed with saturated brine (100 mL), dried over anhydrous sodium sulfate and filtered. The filtrate was concentrated under reduced pressure to obtain a crude product which was dissolved in tetrahydrofuran (40 mL), hydrochloric acid aqueous solution (40 mL, 2 N) was added dropwise thereto and thus the obtained mixture was stirred at room temperature for 1.5 hours after the addition was completed. TLC test was used to monitor the reaction until the reaction was completed. Then the reaction was quenched by dropwise adding saturated solution of sodium bicarbonate. The thus obtained mixture was concentrated under reduced pressure, the aqueous phase was extracted with ethyl acetate (100 mL×4), and the resultant organic phases were combined, washed with saturated brine (100 mL), dried over anhydrous sodium sulfate, and filtered. The filtrate was concentrated under reduced pressure and purified by silica gel column chromatography (eluent: ethyl acetate/petroleum ether (v/v)=1/3). The obtained racemate was separated and purified by preparative liquid chromatography (AD column, n-hexane: isopropanol =95:5, 214 nm, 25°C, retention time: 9.877 minutes) to obtain titled compound 8a (colorless oil, 5.8 g, with a yield of 48.0%).

¹H NMR (400 MHz, DMSO) δ 7.19 (dt, J = 10.4, 1.9 Hz, 1H), 5.93 (dd, J = 10.4, 2.8 Hz, 1H), 4.58 (s, 1H), 2.44-2.38 (m, 1H), 2.38-2.29 (m, 2H), 2.08-1.96 (m, 1H), 1.62 (tdd, J = 12.9, 9.4, 5.0 Hz, 1H).

LC-MS m/z (ESI)= 161.1 [M+1].

### Step 2:

### (1S,4S)-4-(2-hydroxypropan-2-yl-1,1,1,3,3,3-d6)-1-(methyl-d3)cyclohex-2-en-1-ol 7d

Under nitrogen gas atmosphere, deuterated methyl magnesium iodide (34 mL, 33.7 mmol, 1.0 N) was added dropwise to anhydrous lithium chloride (1.4 g, 33.7 mmol) in tetrahydrofuran (20 mL) at 0°C, and the mixture was stirred for 0.5 hour after the addition was completed. Compound 8a (1.8 g, 11.2 mmol) in tetrahydrofuran (5 mL) was added dropwise to the mixture and further reacted under stirring for 0.5 hour after the addition was completed. TLC test was used to monitor the reaction until the reaction was completed. Saturated solution of ammonium chloride (20 mL) was added dropwise to quench the reaction, and the aqueous phase was extracted with ethyl acetate (50 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography to obtain titled compound 8b (white solid, 1.2 g, with a yield of 60.0%).

¹H NMR (400 MHz, DMSO) δ 5.61 (d, J = 10.4 Hz, 1H), 5.52 (d, J = 10.6 Hz, 1H), 4.38 (s, 1H), 4.17 (s, 1H), 2.00 (ddd, J = 10.8, 5.1, 2.4 Hz, 1H), 1.73-1.65 (m, 2H), 1.57-1.48 (m, 1H), 1.21 (dd, J = 10.1, 6.8 Hz, 1H).

### Step 3:

### (1'S,2'S)-2'-(2-hydroxypropan-2-yl-1,1,1,3,3,3-d6)-5'-(methyl-d3)-4-pentyl-1',2',3',4'-tetrahydro-[1,1'-biphenyl]-2,6-diol 8c

Under nitrogen gas atmosphere, L-(-) camphorsulfonic acid (65 mg, 0.28 mmol) was added dropwise to compound 8b (500 mg, 2.79 mmol), 3,5-dihydroxypentylbenzene (0.75 g, 4.18 mmol) and 4A molecular sieve (1.5 g) in dichloromethane (10 mL). After the addition was completed, the resultant mixture was reacted under stirring for 1.5 hours at room temperature. TLC test was used to monitor the reaction until the reaction was completed. Saturated solution of sodium bicarbonate (10 mL) was added dropwise to quench the reaction, and the aqueous phase was extracted with dichloromethane (30 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/10) to obtain titled compound 8c (white solid, 390 mg, with a yield of 40.0%).

¹H NMR (400 MHz, DMSO) δ 8.73 (d, J = 110.9 Hz, 2H), 6.02 (d, J = 11.4 Hz, 2H), 4.88 (s, 1H), 3.66 (d, J = 5.5 Hz, 2H), 2.33 (dt, J = 15.2, 8.7 Hz, 3H), 2.13-2.06 (m, 1H), 2.02 (s, 1H), 1.83 (d, J = 16.6 Hz, 1H), 1.46 (dd, J = 14.6, 7.2 Hz, 2H), 1.25 (ddd, J = 11.6, 11.1, 5.5 Hz, 5H), 0.85 (t, J = 7.0 Hz, 3H).

LC-MS m/z (ESI)= 342.3 [M+1].

### Step 4:

### (1'S,2'S)-5'-(methyl-d3)-4-pentyl-2'-(prop-1-en-2-yl-d5)-1',2',3',4'-tetrahydro-[1,1'-biphenyl]-2,6-diol (compound 8)

Under nitrogen gas atmosphere, Burgess reagent (400 mg, 1.70 mmol) was added into compound 8c (390 mg, 1.13 mmol) in tetrahydrofuran (8 mL) at 0°C and the resultant mixture was warmed to room temperature and reacted under stirring for 20 minutes. TLC test was used to monitor the reaction until the reaction was completed. Saturated solution of sodium bicarbonate (10 mL) was added dropwise to quench the reaction, and the aqueous phase was extracted with ethyl acetate (30 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/30) to obtain titled compound 8 (white solid, 81 mg; HPLC: 95%; with a yield of 23.0%).

¹H NMR (400 MHz, DMSO) δ 8.64 (s, 2H), 6.01 (s, 2H), 5.08 (s, 1H), 3.82 (d, J = 10.5 Hz, 1H), 3.12-2.95 (m, 1H), 2.32-2.25 (m, 2H), 2.15-2.04 (m, 1H), 1.91 (d, J = 16.8 Hz, 1H), 1.71-1.55 (m, 2H), 1.47 (dt, J = 14.8, 7.4 Hz, 2H), 1.31-1.22 (m, 4H), 0.86 (t, J = 7.0 Hz, 3H).

LC-MS m/z (ESI)= 323.3 [M+1].

### Example 9

### (1'R,2'R)-4-heptyl-5'-(methyl-d3)-2'-(prop-1-en-2-yl)-1',2',3',4'-tetrahydro-[1,1'-biphenyl]-2,6-diol (compound 9)

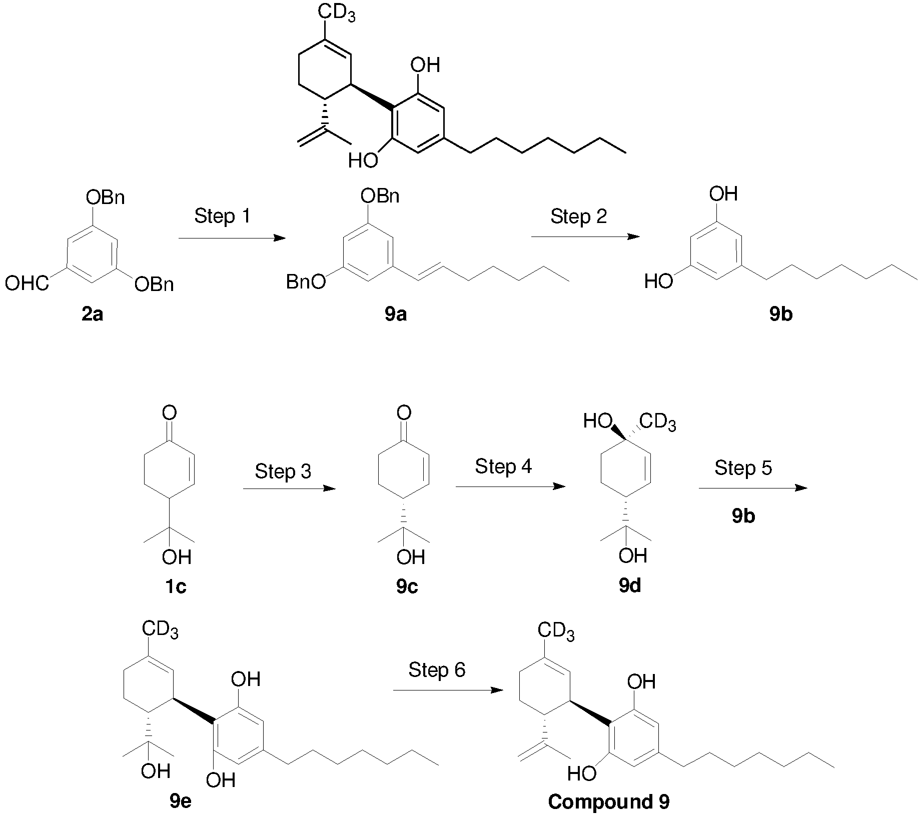

### Step 1:

### (E)-(((5-(hept-1-en-1-yl)-1,3-phenylene)bis(oxy))bis(methylene))dibenzene 9a

Under nitrogen gas atmosphere, sodium bis(trimethylsilyl)amide (113 mL, 226.1 mmol, 2.0 N) was added dropwise to n-bromohexyltriphenylphosphine (100.0 g, 235.0 mmol) in tetrahydrofuran (500 mL). After the addition was completed, the resultant mixture was warmed to 10°C and stirred for 0.5 hour. 3,5-dibenzyloxybenzaldehyde 2a (30.0 g, 94.0 mmol) in tetrahydrofuran (100 mL) was added dropwise thereto, followed by stirring for 1.5 hour. TLC test was used to monitor the reaction until the reaction was completed. Saturated solution of ammonium chloride (300 mL) was added dropwise to quench the reaction and the resultant mixture was subjected to liquid separation. The aqueous phase was extracted with petroleum ether (300 mL×2), and the resultant organic phases were combined and washed with saturated brine (200 mL), dried over anhydrous sodium sulfate, and filtered. The filtrate was concentrated under reduced pressure and purified by silica-gel column chromatography (eluent: ethyl acetate/petroleum ether (v/v)=1/10) to obtain titled compound 9a (colorless oil, 47.0 g, with a yield of 99.0%).

¹H NMR (400 MHz, DMSO) δ 7.44-7.30 (m, 11H), 6.60 (t, J = 2.1 Hz, 1H), 6.53 (t, J = 7.6 Hz, 2H), 6.32 (d, J = 12.0 Hz, 1H), 5.60 (dt, J = 11.7, 7.2 Hz, 1H), 5.08 (s, 4H), 2.24-2.14 (m, 2H), 1.35 (dd, J = 14.2, 7.2 Hz, 2H), 1.27-1.18 (m, 4H), 0.84 (t, J = 6.9 Hz, 3H).

LC-MS m/z (ESI)= 387.3 [M+1].

### Step 2:

### 5-heptylbenzene-1,3-diol 9b

Palladium-carbon (3.2 g, 10%) was added to compound 9a (32.0 g, 83.0 mmol) in anhydrous methanol (150 mL) and ethyl acetate (150 mL) to perform hydrogenation reaction for 15 hour. TLC test was used to monitor the reaction until the reaction was completed. After filtration, the filtrate was concentrated under reduced pressure and purified by silica gel column chromatography (eluent: ethyl acetate/petroleum ether (v/v)=1/3) to obtain titled compound 9b (white solid, 17.4 g, with a yield of 99.0%).

¹H NMR (400 MHz, DMSO) δ 8.98 (s, 2H), 6.02 (s, 3H), 2.42-2.30 (m, 2H), 1.47 (dd, J = 13.9, 6.9 Hz, 2H), 1.26 (d, J = 5.8 Hz, 8H), 0.86 (t, J = 6.9 Hz, 3H).

LC-MS m/z (ESI)= 209.1 [M+1].

### Step 3:

### (R)-4-(2-hydroxypropan-2-yl)cyclohex-2-en-1-one 9c

The racemate 1c was separated and purified by preparative liquid chromatography (AD column, n-hexane: isopropanol =95:5, 214 nm, 25°C, retention time: 8.638 minutes) to obtain title chiral compound 9c.

### Step 4:

### (1R,4R)-4-(2-hydroxypropan-2-yl)-1-(methyl-d3)cyclohex-2-en-1-ol 9d

Under nitrogen gas atmosphere, deuterated methyl magnesium iodide (34 mL, 33.7 mmol, 1.0 N) was added dropwise to anhydrous lithium chloride (1.4 g, 33.7 mmol) in tetrahydrofuran (20 mL) at 0°C, and the mixture was stirred for 0.5 hour after the addition was completed. Compound 9c (1.8 g, 11.2 mmol) in tetrahydrofuran (5 mL) was added dropwise to the mixture and further reacted under stirring for 0.5 hour after the addition was completed. TLC test was used to monitor the reaction until the reaction was completed. Saturated solution of ammonium chloride (20 mL) was added dropwise to quench the reaction, and the aqueous phase was extracted with ethyl acetate (50 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography to obtain titled compound 9d (white solid, 1.2 g, with a yield of 60.0%).

¹H NMR (400 MHz, DMSO) δ 5.61 (d, J = 10.4 Hz, 1H), 5.52 (d, J = 10.6 Hz, 1H), 4.38 (s, 1H), 4.17 (s, 1H), 2.00 (ddd, J = 10.8, 5.1, 2.4 Hz, 1H), 1.73-1.65 (m, 2H), 1.57-1.48 (m, 1H), 1.21 (dd, J = 10.1, 6.8 Hz, 1H), 0.99 (s, 3H), 0.73 (s, 3H).

LC-MS m/z (ESI) = 174.6 [M+1].

### Step 5:

### (1'R,2'R)-4-heptyl-2'-(2-hydroxypropan-2-yl)-5'-(methyl-d3)-1',2',3',4'-tetrahydro-[1,1'-biphenyl]-2,6-diol 9e

Under nitrogen gas atmosphere, L-(-) camphorsulfonic acid (67 mg, 0.29 mmol) was added to compound 9b (1.6 g, 8.67 mmol), compound 9d (500 mg, 2.89 mmol) and 4A molecular sieve (1.5 g) in dichloromethane (10 mL). After the addition was completed, the resultant mixture was reacted under stirring for 1.5 hours at room temperature. TLC test was used to monitor the reaction until the reaction was completed. Saturated solution of sodium bicarbonate (10 mL) was added dropwise to quench the reaction, and the aqueous phase was extracted with dichloromethane (30 mL×2). The resultant organic phases were combined and washed with saturated brine (30 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/6) to obtain titled compound 9e (colorless oil, 410 mg, with a yield of 41.0%).

¹H NMR (400 MHz, DMSO)δ 8.67 (dd, J = 96.6, 46.4 Hz, 2H), 6.03 (s, 2H), 4.89 (s, 1H), 3.66 (d, J = 10.8 Hz, 2H), 2.40-2.28 (m, 3H), 2.05 (ddd, J = 14.8, 5.7, 1.9 Hz, 2H), 1.83 (d, J = 16.5 Hz, 1H), 1.47 (dd, J = 14.1, 7.2 Hz, 2H), 1.28 (d, J = 24.3 Hz, 9H), 0.97 (s, 3H), 0.85 (t, J = 6.8 Hz, 3H), 0.80 (s, 3H).

LC-MS m/z (ESI)= 364.3 [M+1].

### Step 6:

### (1'R,2'R)-4-heptyl-5'-(methyl-d3)-2'-(prop-1-en-2-yl)-1',2',3',4'-tetrahydro-[1,1'-biphenyl]-2,6-diol (compound 9)

Under nitrogen gas atmosphere, Burgess reagent (540 mg, 2.26 mmol) was added into compound 9e (410 mg, 1.13 mmol) in tetrahydrofuran (5 mL) at 0°C and the resultant mixture was warmed to room temperature and reacted under stirring for 30 minutes. TLC test was used to monitor the reaction until the reaction was completed. Saturated solution of sodium bicarbonate (10 mL) was added dropwise to quench the reaction, and the aqueous phase was extracted with ethyl acetate (30 mL×3). The resultant organic phases were combined and washed with saturated brine (30 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/30) to obtain titled compound 9 (white solid, 245 mg; HPLC: 99.5%; with a yield of 63%).

¹H NMR (400 MHz, DMSO) δ 8.63 (s, 2H), 6.01 (s, 2H), 5.08 (s, 1H), 4.49 (d, J = 2.5 Hz, 1H), 4.40 (d, J = 1.0 Hz, 1H), 3.82 (d, J = 10.5 Hz, 1H), 3.08-2.95 (m, 1H), 2.30 (dd, J = 14.0, 6.5 Hz, 2H), 2.15-2.04 (m, 1H), 1.91 (d, J = 16.6 Hz, 1H), 1.72-1.63 (m, 1H), 1.58 (s, 3H), 1.50-1.42 (m, 2H), 1.26 (d, J = 5.7 Hz, 9H), 0.85 (t, J = 6.8 Hz, 3H).

LC-MS m/z (ESI)= 346.3 [M+1].

### Example 10

### (1'S,2'S)-4-heptyl-5'-(methyl-d3)-2'-(prop-1-en-2-yl)-1',2',3',4'-tetrahydro-[1,1'-biphenyl]-2,6-diol (compound 10)

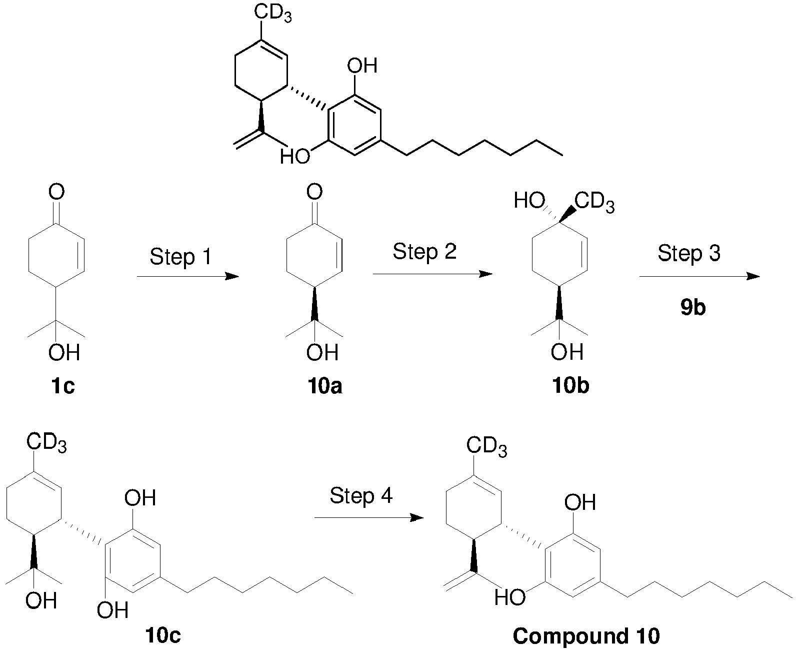

### Step 1:

### (S)-4-(2-hydroxypropan-2-yl)cyclohex-2-en-1-one 10a

The racemate 1c was separated and purified by preparative liquid chromatography (AD column, n-hexane: isopropanol =95:5, 214 nm, 25°C, retention time: 9.894 minutes) to obtain the title chiral compound 10a

### Step 2:

### (1S,4S)-4-(2-hydroxypropan-2-yl)-1-(methyl-d3)cyclohex-2-en-1-ol 10b

Under nitrogen gas atmosphere, deuterated methyl magnesium iodide (34 mL, 33.7 mmol, 1.0 N) was added dropwise to anhydrous lithium chloride (1.4 g, 33.7 mmol) in tetrahydrofuran (20 mL) at 0°C, and the mixture was stirred for 0.5 hour after the addition was completed. Compound 10a (1.8 g, 11.2 mmol) in tetrahydrofuran (5 mL) was added dropwise to the mixture and further reacted under stirring for 0.5 hour after the addition was completed. TLC test was used to monitor the reaction until the reaction was completed. Saturated solution of ammonium chloride (20 mL) was added dropwise to quench the reaction, and the aqueous phase was extracted with ethyl acetate (50 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography to obtain titled compound 10 (white solid, 1.2 g, with a yield of 60.0%).

¹H NMR (400 MHz, DMSO) δ 5.61 (d, J = 10.4 Hz, 1H), 5.52 (d, J = 10.6 Hz, 1H), 4.38 (s, 1H), 4.17 (s, 1H), 2.00 (ddd, J = 10.8, 5.1, 2.4 Hz, 1H), 1.73-1.65 (m, 2H), 1.57-1.48 (m, 1H), 1.21 (dd, J = 10.1, 6.8 Hz, 1H), 0.99 (s, 3H), 0.73 (s, 3H).

LC-MS m/z (ESI) = 174.6 [M+1].

### Step 3:

### (1'S,2'S)-4-heptyl-2'-(2-hydroxypropan-2-yl)-5'-(methyl-d3)-1',2',3',4'-tetrahydro-[1,1'-biphenyl]-2,6-diol 10c

Under nitrogen gas atmosphere, L-(-) camphorsulfonic acid (67 mg, 0.29 mmol) was added to compound 10b (500 mg, 2.89 mmol), 5-heptylbenzene-1,3-diol 9d (1.6 g, 8.67 mmol) and 4A molecular sieve (1.5 g) in dichloromethane (10 mL). After the addition was completed, the resultant mixture was reacted under stirring for 1.5 hours at room temperature. TLC test was used to monitor the reaction until the reaction was completed. Saturated solution of sodium bicarbonate (10 mL) was added dropwise to quench the reaction, and the aqueous phase was extracted with dichloromethane (30 mL×2). The resultant organic phases were combined and washed with saturated brine (30 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/6) to obtain titled compound 10c (colorless oil, 400 mg, with a yield of 40.0%).

¹H NMR (400 MHz, DMSO) δ 8.67 (dd, J = 101.3, 50.3 Hz, 2H), 6.03 (s, 2H), 4.89 (s, 1H), 3.66 (d, J = 11.0 Hz, 2H), 2.39-2.27 (m, 3H), 2.14-1.97 (m, 2H), 1.83 (d, J = 16.5 Hz, 1H), 1.47 (dd, J = 14.2, 7.2 Hz, 2H), 1.30-1.18 (m, 9H), 0.97 (s, 3H), 0.85 (t, J = 6.9 Hz, 3H), 0.80 (s, 3H).

LC-MS m/z (ESI)= 364.3 [M+1].

### Step 4:

### (1'S,2'S)-4-heptyl-5'-(methyl-d3)-2'-(prop-1-en-2-yl)-1',2',3',4'-tetrahydro-[1,1'-biphenyl]-2,6-diol (compound 10)

Under nitrogen gas atmosphere, 4A molecular sieve (1.5 g), triethylamine (230 mg, 2.20 mmol) and Burgess reagent (524 mg, 2.26 mmol) were respectively added into compound 10c (400 mg, 1.10 mmol) in tetrahydrofuran (5 mL) at 0°C and the resultant mixture was warmed to room temperature and reacted under stirring for 30 minutes. TLC test was used to monitor the reaction until the reaction was completed. Saturated solution of sodium bicarbonate (10 mL) was added dropwise to quench the reaction, and the aqueous phase was extracted with ethyl acetate (30 mL×3). The resultant organic phases were combined and washed with saturated brine (30 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/30) to obtain titled compound 10 (white solid, 220 mg; HPLC: 99.5%; with a yield of 57%).

¹H NMR (400 MHz, DMSO) δ 8.63 (s, 2H), 6.01 (s, 2H), 5.08 (s, 1H), 4.49 (d, J = 2.5 Hz, 1H), 4.40 (d, J = 1.0 Hz, 1H), 3.82 (d, J = 10.5 Hz, 1H), 3.09-2.96 (m, 1H), 2.34-2.21 (m, 2H), 2.16-2.04 (m, 1H), 1.99-1.85 (m, 1H), 1.71-1.64 (m, 1H), 1.58 (s, 3H), 1.51-1.42 (m, 2H), 1.26 (d, J = 5.6 Hz, 9H), 0.85 (t, J = 6.8 Hz, 3H).

LC-MS m/z (ESI) = 346.3 [M+1].

### Example 11

### 4-(heptyl-1,1-d2)-5'-(methyl-d3)-2'-(prop-1-en-2-yl)-1',2',3',4'-tetrahydro-[1,1'-biphenyl]-2,6-diol (compound 11)

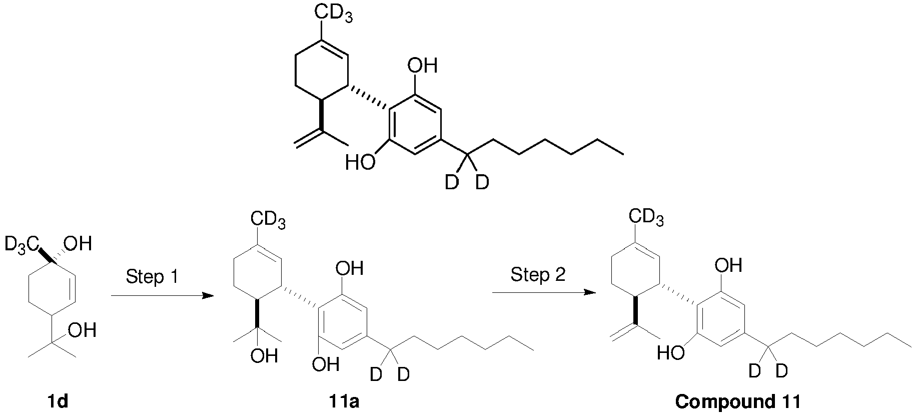

### Step 1:

### 4-(heptyl-1,1-d2)-2'-(2-hydroxypropan-2-yl)-5'-(methyl-d3)-1',2',3',4'-tetrahydro-[1,1'-biphenyl]-2,6-diol 11a

Under nitrogen gas atmosphere, L-(-) camphorsulfonic acid (67 mg, 0.29 mmol) was added to compound 1d (500 mg, 2.89 mmol), 5-(heptyl-1,1-d2)phenyl-1,3-diol 9d (1.6 g, 8.67 mmol) and 4A molecular sieve (1.5 g) in dichloromethane (10 mL). After the addition was completed, the resultant mixture was reacted under stirring for 1.5 hours at room temperature. TLC test was used to monitor the reaction until the reaction was completed. Saturated solution of sodium bicarbonate (10 mL) was added dropwise to quench the reaction, and the aqueous phase was extracted with dichloromethane (30 mL×2). The resultant organic phases were combined and washed with saturated brine (30 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/6) to obtain titled compound 11a (colorless oil, 400 mg, with a yield of 40.0%).

¹H NMR (400 MHz, DMSO) δ 8.67 (dd, 2H), 6.03 (s, 2H), 4.89 (s, 1H), 3.66 (d, 2H), 2.39-2.27 (m, 1H), 2.14-1.97 (m, 2H), 1.83 (d, 1H), 1.47 (dd, 2H), 1.30-1.18 (m, 9H), 0.97 (s, 3H), 0.85 (t, 3H), 0.80 (s, 3H).

LC-MS m/z (ESI) = 366.3 [M+1].

### Step 2:

### 4-(heptyl-1,1-d2)-5'-(methyl-d3)-2'-(prop-1-en-2-yl)-1',2',3',4'-tetrahydro-[1,1'-biphenyl]-2,6-diol (compound 11)

Under nitrogen gas atmosphere, 4A molecular sieve (1.5 g), triethylamine (230 mg, 2.20 mmol) and Burgess reagent (524 mg, 2.26 mmol) were respectively added into compound 11a (400 mg, 1.10 mmol) in tetrahydrofuran (5 mL) at 0°C and the resultant mixture was warmed to room temperature and reacted under stirring for 30 minutes. TLC test was used to monitor the reaction until the reaction was completed. Saturated solution of sodium bicarbonate (10 mL) was added dropwise to quench the reaction, and the aqueous phase was extracted with ethyl acetate (30 mL×3). The resultant organic phases were combined and washed with saturated brine (30 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/30) to obtain titled compound 11 (white solid, 141 mg; HPLC: 99.5%; with a yield of 37%).

¹H NMR (400 MHz, DMSO) δ 8.63 (s, 2H), 6.01 (s, 2H), 5.08 (s, 1H), 4.49 (d, 1H), 4.40 (d, 1H), 3.82 (d, 1H), 3.09-2.96 (m, 1H), 2.16-2.04 (m, 1H), 1.99-1.85 (m, 1H), 1.71-1.64 (m, 1H), 1.58 (s, 3H), 1.51-1.42 (m, 2H), 1.26 (d, 9H), 0.85 (t, 3H).

LC-MS m/z (ESI) = 348.3 [M+1].

### Example 12

### (1'R,2'R)-5'-(methyl-d3)-4-pentyl-2'-(prop-1-en-2-yl)-1',2',3',4'-tetrahydro-[1,1'-biphenyl]-4'-d-2,6-diol (compound 12)

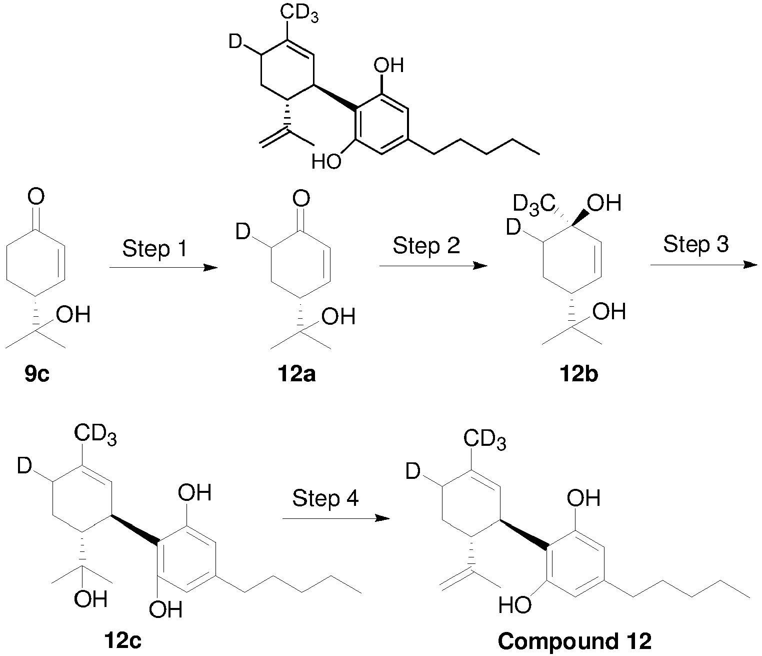

### Step 1:

### (4R)-4-(2-hydroxypropan-2-yl)cyclohex-2-en-1-one-6-d 9c

Under nitrogen gas atmosphere, heavy water (7 mL, 350.0 mmol) and potassium carbonate (54 mg, 0.4 mmol) were added into (R)-4-(2-hydroxypropan-2-yl)cyclohex-2-en-1-one 9c (3.0 g, 19.5 mmol) in tetrahydrofuran (14 mL) and deuterated methanol (14 mL), followed by stirring at room temperature for 48 hours. LC-MS was used to monitor the reaction until the reaction was completed. The reaction mixture was concentrated under reduced pressure, and the aqueous phase was extracted with ethyl acetate (30 mL×3). The resultant organic phases were combined and washed with saturated brine (30 mL), dried over anhydrous sodium sulfate, and filtered. The filtrate was concentrated under reduced pressure and purified by silica-gel column chromatography (eluent: ethyl acetate/petroleum ether (v/v)=1/1) to obtain titled compound 12a (colorless oil, 2.4g; with a yield of 79.0%).

¹H NMR (400 MHz, DMSO) δ 7.19 (dt, J = 10.3, 1.9 Hz, 1H), 5.99-5.88 (m, 1H), 4.60 (s, 1H), 2.44-2.38 (m, 1H), 2.32 (d, J = 10.7 Hz, 1H), 2.06-1.99 (m, 1H), 1.67-1.56 (m, 1H), 1.15 (s, 3H), 1.05 (s, 3H).

LC-MS m/z (ESI)= 178.1 [M+Na].

### Step 2:

### (1R,4R)-4-(2-hydroxypropan-2-yl)-1-(methyl-d3)cyclohex-2-en-6-d-1-ol 12b

Under nitrogen gas atmosphere, deuterated methyl magnesium iodide (14 mL, 13.5 mol, 1.0 N) was added dropwise to anhydrous lithium chloride (570 mg, 13.5 mmol) in tetrahydrofuran (10 mL) at 0°C, and the mixture was stirred for 0.5 hour after the addition was completed. Compound 12a (700 mg, 4.0 mmol) in tetrahydrofuran (5 mL) was added dropwise to the mixture and further reacted under stirring for 0.5 hour after the addition was completed. TLC test was used to monitor the reaction until the reaction was completed. Saturated solution of ammonium chloride (20 mL) was added dropwise to quench the reaction, and the aqueous phase was extracted with ethyl acetate (30 mL×3). The resultant organic phase was combined and washed with saturated brine (50 mL), dried over anhydrous sodium sulfate, and filtered. The filtrate was concentrated under reduced pressure to obtain a crude product which was purified by silica-gel column chromatography to obtain titled compound 12b (white solid, 430 mg, with a yield of 55.0%).

¹H NMR (400 MHz, DMSO) δ 5.66-5.58 (m, 1H), 5.55-5.46 (m, 1H), 4.40 (d, J = 1.8 Hz, 1H), 4.21 (s, 1H), 1.99 (ddd, J = 10.4, 5.4, 2.4 Hz, 1H), 1.68 (dd, J = 12.9, 5.5 Hz, 2H), 1.24-1.20 (m, 1H), 1.03 (s, 3H), 0.97 (s, 3H).

### Step 3:

### (1'R,2'R)-2'-(2-hydroxypropan-2-yl)-5'-(methyl-d3)-4-pentyl-1',2',3',4'-tetrahydro-[1,1'-biphenyl]-4'-d-2,6-diol 12c

Under nitrogen gas atmosphere, L-(-) camphorsulfonic acid (53 mg, 0.23 mmol) was added to compound 12b (400 mg, 2.29 mmol), 3,5-dihydroxypentylbenzene (621 mg, 3.44 mmol) and 4A molecular sieve (1.2 g) in dichloromethane (10 mL), and after the addition was completed, the resultant mixture was reacted under stirring for 1.5 hours at room temperature. TLC test was used to monitor the reaction until the reaction was completed. Saturated solution of sodium bicarbonate (10 mL) was added dropwise to quench the reaction, and the aqueous phase was extracted with dichloromethane (30 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/6) to obtain titled compound 12c (white solid, 270 mg, with a yield of 35.0%).

¹H NMR (400 MHz, DMSO) δ 9.02-8.43 (m, 2H), 6.03 (s, 2H), 4.88 (d, J = 2.3 Hz, 1H), 3.66 (d, J = 11.0 Hz, 2H), 2.39-2.28 (m, 3H), 2.09 (d, J = 12.6 Hz, 1H), 1.81 (s, 1H), 1.46 (dt, J = 14.6, 7.4 Hz, 2H), 1.32-1.22 (m, 6H), 0.97 (s, 3H), 0.85 (t, J = 7.0 Hz, 3H), 0.79 (s, 3H).

LC-MS m/z (ESI) = 337.3 [M+1].

### Step 4:

### (1'R,2'R)-5'-(methyl-d3)-4-pentyl-2'-(prop-1-en-2-yl)-1',2',3',4'-tetrahydro-[1,1'-biphenyl]-4'-d-2,6-diol (compound 12)

Under nitrogen gas atmosphere, Burgess reagent (284 mg, 1.12 mmol) was added into compound 12c (270 mg, 0.79 mmol) in tetrahydrofuran (6 mL) at 0°C and the resultant mixture was warmed to room temperature and reacted under stirring for 30 minutes. TLC test was used to monitor the reaction until the reaction was completed. Saturated solution of sodium bicarbonate (10 mL) was added dropwise to quench the reaction, and the aqueous phase was extracted with ethyl acetate (30 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/30) to obtain titled compound 12 (white solid, 73 mg; HPLC: 99%; with a yield of 28.0%).

¹H NMR (400 MHz, DMSO) δ 8.64 (s, 2H), 6.01 (s, 2H), 5.08 (s, 1H), 4.49 (d, J = 2.4 Hz, 1H), 4.40 (s, 1H), 3.82 (d, J = 10.4 Hz, 1H), 3.08-2.95 (m, 1H), 2.34-2.25 (m, 2H), 1.89 (s, 1H), 1.71-1.61 (m, 1H), 1.58 (s, 3H), 1.47 (dt, J = 14.7, 7.4 Hz, 2H), 1.36-1.20 (m, 5H), 0.86 (t, J = 7.0 Hz, 3H).

LC-MS m/z (ESI) = 319.2 [M+1].

### Example 13

### (1'R,2'R)-5'-(methyl-d3)-4-pentyl-2'-(prop-1-en-2-yl)-1',2',3',4'-tetrahydro-[1,1'-biphenyl]-4',4'-d2-2,6-diol (compound 13)

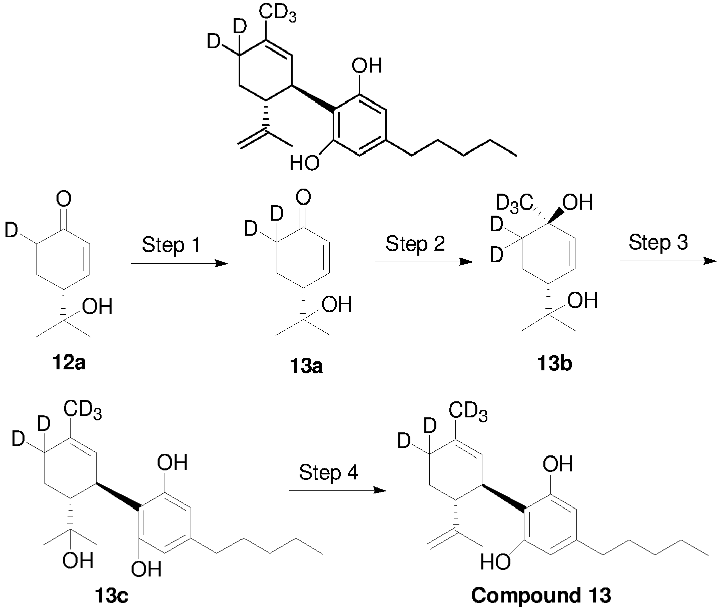

### Step 1:

### (R)-4-(2-hydroxypropan-2-yl)cyclohex-2-en-1-one-6,6-d2 13a

Under nitrogen gas atmosphere, lithium diisopropylamide (14 mL, 27.0 mmol) was added dropwise into (4R)-4-(2-hydroxypropan-2-yl)cyclohex-2-en-1-one-6-d 12a (2.0 g, 12.9 mmol) in tetrahydrofuran (20 mL) at -70°C. After the addition was completed, the mixture was stirred for reaction for 0.5 hours. Then heavy water (5 mL, 250.0 mmol) was added dropwise thereto and the mixture was warmed to room temperature and stirred for 20 minutes. The aqueous phase was extracted with ethyl acetate (30 mL×3). The resultant organic phases were combined and washed with saturated brine (30 mL), dried over anhydrous sodium sulfate, and filtered. The filtrate was concentrated under reduced pressure and purified by silica-gel column chromatography (eluent: ethyl acetate/petroleum ether (v/v)=1/1) to obtain titled compound 13a (colorless oil, 1.4g; with a yield of 70.0%).

¹H NMR (400 MHz, DMSO) δ 7.19 (dt, J = 10.3, 1.9 Hz, 1H), 5.99-5.88 (m, 1H), 4.60 (s, 1H), 2.32 (d, J = 10.7 Hz, 1H), 2.06-1.99 (m, 1H), 1.67-1.56 (m, 1H), 1.15 (s, 3H), 1.05 (s, 3H).

LC-MS m/z (ESI) = 179.1 [M+Na].

### Step 2:

### (1R,4R)-4-(2-hydroxypropan-2-yl)-1-(methyl-d3)cyclohex-2-en-6,6-d2-1-ol 13b

Under nitrogen gas atmosphere, deuterated methyl magnesium iodide (12 mL, 11.5 mmol, 1.0 N) was added dropwise to anhydrous lithium chloride (488 mg, 11.5 mmol) in tetrahydrofuran (10 mL) at 0°C, and the mixture was stirred for 0.5 hour after the addition was completed. Compound 13a (600 mg, 3.8 mmol) in tetrahydrofuran (5 mL) was added dropwise to the mixture and further reacted under stirring for 0.5 hour after the addition was completed. TLC test was used to monitor the reaction until the reaction was completed. Saturated solution of ammonium chloride (20 mL) was added dropwise to quench the reaction, and the aqueous phase was extracted with ethyl acetate (30 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography to obtain titled compound 13b (white solid, 410 mg, with a yield of 60.0%).

¹H NMR (400 MHz, DMSO)δ 5.66-5.58 (m, 1H), 5.55-5.46 (m, 1H), 4.40 (d, J = 1.8 Hz, 1H), 4.21 (s, 1H), 1.99 (ddd, J = 10.4, 5.4, 2.4 Hz, 1H), 1.68 (dd, J = 12.9, 5.5 Hz, 1H), 1.24-1.20 (m, 1H), 1.03 (s, 3H), 0.97 (s, 3H).

### Step 3:

### (1'R,2'R)-2'-(2-hydroxypropan-2-yl)-5'-(methyl-d3)-4-pentyl-1',2',3',4'-tetrahydro-[1,1'-biphenyl]-4',4'-d2-2,6-diol 13c

Under nitrogen gas atmosphere, L-(-) camphorsulfonic acid (53 mg, 0.23 mmol) was added to compound 13b (400 mg, 2.29 mmol), 3,5-dihydroxypentylbenzene (621 mg, 3.44 mmol) and 4A molecular sieve (1.2 g) in dichloromethane (10 mL). After the addition was completed, the resultant mixture was reacted under stirring for 1.5 hours at room temperature. TLC test was used to monitor the reaction until the reaction was completed. Saturated solution of sodium bicarbonate (10 mL) was added dropwise to quench the reaction, and the aqueous phase was extracted with dichloromethane (30 mL×3). The organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/6) to obtain titled compound 13c (white solid, 250 mg, with a yield of 32.5%).

¹H NMR (400 MHz, DMSO) δ 9.02-8.43 (m, 2H), 6.03 (s, 2H), 4.88 (d, J = 2.3 Hz, 1H), 3.66 (d, J = 11.0 Hz, 2H), 2.39-2.28 (m, 3H), 1.81 (s, 1H), 1.46 (dt, J = 14.6, 7.4 Hz, 2H), 1.32-1.22 (m, 6H), 0.97 (s, 3H), 0.85 (t, J = 7.0 Hz, 3H), 0.79 (s, 3H).

LC-MS m/z (ESI) = 338.3 [M+1].

### Step 4:

### (1'R,2'R)-5'-(methyl-d3)-4-pentyl-2'-(prop-1-en-2-yl)-1',2',3',4'-tetrahydro-[1,1'-biphenyl]-4',4'-d2-2,6-diol (compound 13)

Under nitrogen gas atmosphere, Burgess reagent (264 mg, 1.11 mmol) was added into the compound 13c (250 mg, 0.74 mmol) in tetrahydrofuran (6 mL) at 0°C and the resultant mixture was warmed to room temperature and reacted under stirring for 30 minutes. TLC test was used to monitor the reaction until the reaction was completed. Saturated solution of sodium bicarbonate (10 mL) was added dropwise to quench the reaction, and the aqueous phase was extracted with ethyl acetate (30 mL×3). The resultant organic phases were combined and washed with saturated brine (50 mL), dried over anhydrous sodium sulfate, and filtered. The filtrate was concentrated under reduced pressure to obtain a crude product which was purified by silica-gel column chromatography (eluent: ethyl acetate/petroleum ether (v/v)=1/30) to obtain titled compound 13 (white solid, 55 mg; with a yield of 23.3%).

¹H NMR (400 MHz, DMSO) δ 8.64 (s, 2H), 6.01 (s, 2H), 5.08 (s, 1H), 4.49 (d, J = 2.4 Hz, 1H), 4.40 (s, 1H), 3.82 (d, J = 10.4 Hz, 1H), 3.08-2.95 (m, 1H), 2.34-2.25 (m, 2H), 1.89 (s, 1H), 1.58 (s, 3H), 1.47 (dt, J = 14.7, 7.4 Hz, 2H), 1.36-1.20 (m, 5H), 0.86 (t, J = 7.0 Hz, 3H).

LC-MS m/z (ESI) = 320.2 [M+1].

### Example 14

### 4-(methyl-d3)-methyl-5'-(methyl-d3)-2'-(prop-1-en-2-yl)-1',2',3',4'-tetrahydro-[1,1'-biphenyl]-2,6-diol (compound 14)

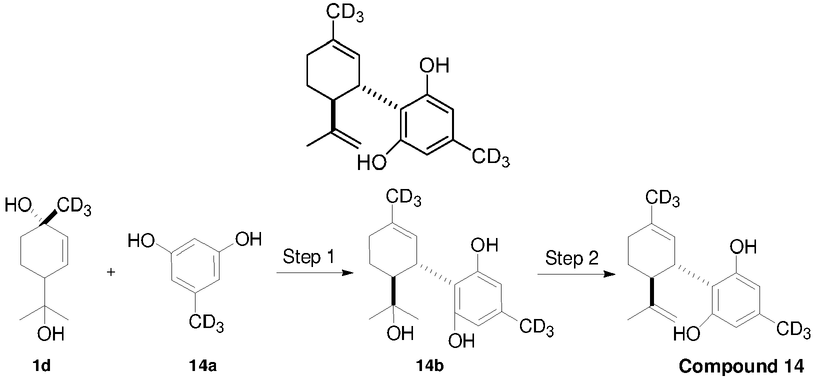

### Step 1:

### 2'-(2-hydroxypropan-2-yl)-4-(methyl-d3)-5'-(methyl-d3)-1',2',3',4'-tetrahydro-[1,1'-biphenyl]-2,6-diol 14b

4-(2-hydroxypropyl-2-yl)-1-(methyl-d3) cyclohex-2-ene-1-ol 1d(500mg, 2.9 mmol), 5-(methyl-d3)benzene-1,3-diol 14a(1080mg, 8.7 mmol) and 4A molecular sieve (1.5g) were dissolved in 10 mL of dichloromethane, the atmosphere in the flask was replaced by nitrogen gas and the thus obtained mixture was stirred for 10 minutes at room temperature. Then, L-camphor sulfonic acid (67.34 mg, 0.29 mmol) was added thereto. The thus obtained mixture was stirred for 1 hour at room temperature, then quenched by adding 20mL of saturated aqueous solution of sodium bicarbonate, and the thus obtained mixture was extracted with ethyl acetate (10mL×4). The resultant organic phases were combined, dried over anhydrous sodium sulfate, and filtered. The filtrate was concentrated to obtain 700mg crude product. Column chromatography separation was performed to obtain 2'-(2-hydroxypropan-2-yl)-4-(methyl-d3)-5'-(methyl-d3)-1',2',3',4'-tetrahydro-[1,1'-biphenyl]-2,6-diol 14b (white solid, 375mg, with a yield of 46%).

¹H NMR (400 MHz, DMSO-d6) δ 9.03 (s, 1H), 8.79 (s, 1H), 6.09 (d, 1H), 5.95 (d, 1H), 4.92 (s, 1H), 3.90 (d, 1H), 2.14 (d, 2H), 1.37-1.21 (m, 2H), 0.99 (s, 3H), 0.91-0.79 (m, 2H), 0.73 (s, 3H).

### Step 2:

### 4-(methyl-d3)-5'-(methyl-d3)-2'-(prop-1-en-2-yl)-1',2',3',4'-tetrahydro-[1,1'-biphenyl]-2,6-diol (compound 14)

2'-(2-hydroxypropan-2-yl)-4-(methyl-d3)-5'-(methyl-d3)-1',2',3',4'-tetrahydro-[1,1'-biphenyl]-2,6-diol 14b (350 mg, 1.25 mmol) was dissolved in 6 mL of tetrahydrofuran, the atmosphere in the flask was replaced by nitrogen gas and the thus obtained system was cooled to 0°C. Then, Burgess reagent (595 mg, 2.5 mmol) was added into the system and followed by warming to room temperature. The reaction was performed for 20 minutes, then quenched by adding 20mL of saturated aqueous solution of sodium bicarbonate, and the thus obtained mixture was extracted with ethyl acetate (10mL×4). The resultant organic phases were combined, dried over anhydrous sodium sulfate, filtered and concentrated to obtain 500mg crude product so as to obtain 4-(methyl-d3)-5'-(methyl-d3)-2'-(prop-1-en-2-yl)-1',2',3',4'-tetrahydro-[1,1'-biphenyl]-2,6-diol (compound 14) (purple solid, 30mg, with a yield of 9.2%).

¹H NMR (400 MHz, Chloroform-d) δ 6.28-6.15 (m, 2H), 5.54 (d, 1H), 4.65 (p, 1H), 4.53-4.37 (m, 1H), 3.61-3.46 (m, 1H), 2.45 (ddd, 1H), 2.22 (ddt, 1H), 2.10 (q, 1H), 1.86-1.69 (m, 2H), 1.56 (t, 3H).

### Example 15

### 5'-(hydroxymethyl-d2)-4-pentyl-2'-(prop-1-en-2-yl)-1',2',3',4'-tetrahydro-[1,1'-biphenyl]-2,6-diol (compound 15)

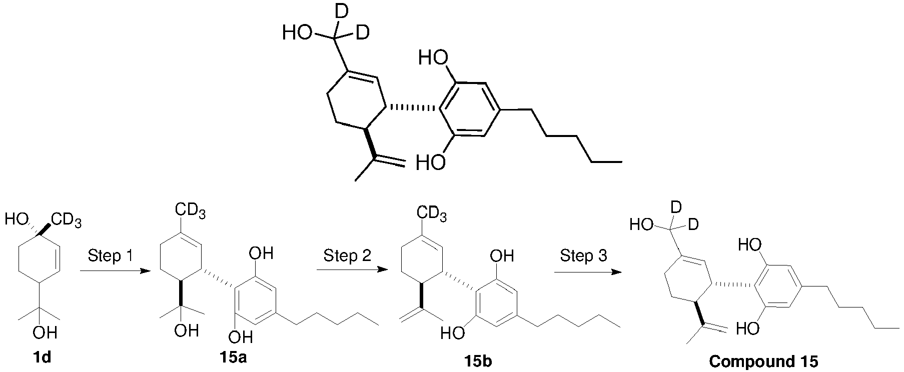

### Step 1:

### 2'-(2-hydroxypropan-2-yl)-5'-(methyl-d3)-4-pentyl-1',2',3',4'-tetrahydro-[1,1'-biphenyl]-2,6-diol 15a

4-(2-hydroxypropyl-2-yl)-1-(methyl-d3) cyclohex-2-ene-1-ol 1d (2.37 g, 13.7 mmol), 3,5-dihydroxyamylbenzene (3.7 g, 20.6 mmol) and 4A molecular sieve (6 g) were dissolved in 30 mL of dichloromethane, the atmosphere in the flask was replaced by nitrogen and the thus obtained mixture was stirred for 20 minutes. Then, L-camphor sulfonic acid (317.8 mg, 1.37 mmol) was added thereto. The thus obtained mixture was stirred for 1 hour at room temperature, then quenched by adding 60mL of saturated aqueous solution of sodium bicarbonate, extracted with ethyl acetate (30mL×4). The resultant organic phases were combined, dried over anhydrous sodium sulfate, and filtered. The filtrate was concentrated to obtain a crude product. After purification by silica gel column chromatography (eluent: ethyl acetate/petroleum ether (v/v)=1/6), titled compound 15a (colorless oil, 1.3 g, with a yield of 28.5%) was obtained.

¹H NMR (400 MHz, DMSO) δ 9.04-8.43 (m, 2H), 6.03 (s, 2H), 4.89 (s, 1H), 3.66 (m, 2H), 2.32 (m, 3H), 2.14-2.06 (m, 1H), 2.02 (m, 1H), 1.88-1.79 (m, 1H), 1.47 (m, 2H), 1.32-1.19 (m, 5H), 0.97 (s, 3H), 0.85 (t, 3H), 0.80 (s, 3H).

LC-MS m/z (ESI) = 336.20 [M+1]

### Step 2:

### 5'-(methyl-d3)-4-pentyl-2'-(prop-1-en-2-yl)-1',2',3',4'-tetrahydro-[1,1'-biphenyl]-2,6-diol 15b

At 0°C, compound 15a (1.3 g, 3.9 mmol), Burgess reagent (1.1 g, 4.7 mmol) and tetrahydrofuran (10 mL) were successively added into the reaction flask, and stirred and reacted for 3 hours under nitrogen gas atmosphere. TLC test was used to monitor the reaction until the reaction was completed. Ethyl acetate (50 mL) was added to the reaction solution, the thus obtained organic phases were washed with saturated brine (40 mL), dried over anhydrous sodium sulfate, and filtered. And the filtrate was concentrated under reduced pressure to obtain a crude product which was purified by silica gel column chromatography (eluent: ethyl acetate/petroleum ether (v/v)=1/15) to obtain titled compound 15b (colorless oil, 794 mg, with a yield of 64.0%).

¹H NMR (400 MHz, DMSO) δ 8.65 (s, 2H), 6.01 (s, 2H), 5.08 (s, 1H), 4.49 (d, 1H), 4.43-4.37 (d, 1H), 3.85-3.78 (m, 1H), 3.08-2.98 (m, 1H), 2.32-2.26 (m, 2H), 2.13-2.04 (m, 1H), 1.95-1.87 (m, 1H), 1.71-1.63 (m, 1H), 1.58 (s, 3H), 1.47 (m, 2H), 1.35-1.22 (m, 5H), 0.86 (t, 3H).

LC-MS m/z (ESI) = 318.20 [M+1].

### Step 3:

### 5'-(hydroxymethyl-d2)-4-pentyl-2'-(prop-1-en-2-yl)-1',2',3',4'-tetrahydro-[1,1'-biphenyl]-2,6-diol (compound 15)

N-hydroxyphthalimide (0.5 mmol) and anhydrous t-BuOOH (1.5 mmol) were added into a mixture of compound 15b (2.5 mmol) and N-Bu₄NI (0.05 mml). The reaction mixture was stirred at 75°C for 1 hour in a sealed condition. Saturated solution of Na₂S₂O₈(10 mL) was added to quench the reaction, and the mixture was extracted with ethyl acetate (3×30 ml). The resultant organic phases were combined, washed with brine (20 ml), dried over anhydrous sodium sulfate, and evaporated under vacuum to remove the solvent. At room temperature, HCl (1.5 mL) was slowly added into a flask containing the above mixture (140 mg, 0.5 mmol) and zinc powder (325 mg, 10 mmol) in methanol (3 mL). The reaction mixture was stirred at room temperature for 20 minutes. The reaction mixture was filtered through diatomite, the solvent was removed under vacuum, and the residue was diluted with water and extracted with EA (3×15 mL). The resultant organic phases were combined, dried over sodium sulfate. After vacuum concentration and purification with silica gel column by using PE/EA to obtain compound 15 (white solid, 51 mg, 31%).

LC-MS m/z (ESI) = 333.2 [M+1].

### Example 22

### 2'-isopropyl-5'-(methyl-d3)-4-pentyl-1',2',3',4'-tetrahydro-[1,1'-biphenyl]-2,6-diol (compound 22)

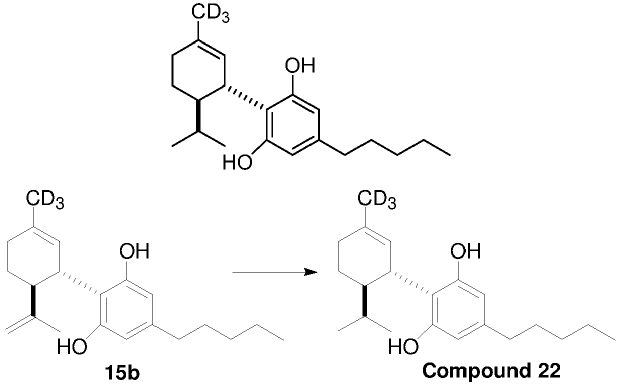

Compound 15b (0.544 g, 1.73 mmol) was dissolved in ethyl acetate (10 ml) at 10 psi, and hydrogenated in the presence of platinum (0.021 g) at 10 psi for 30 minutes. The resultant mixture was purified by silica gel chromatography using petroleum ether and ether as eluents to obtain compound 22 (white solid, 534 mg, 98%).

LC-MS m/z (ESI) = 320.3 [M+1].

### Example 24

### 2',6'-dimethoxy-5-(methyl-d3)-4'-pentyl-2-(prop-1-en-2-yl)-1,2,3,4-tetrahydro-1,1'-biphenyl (compound 24)

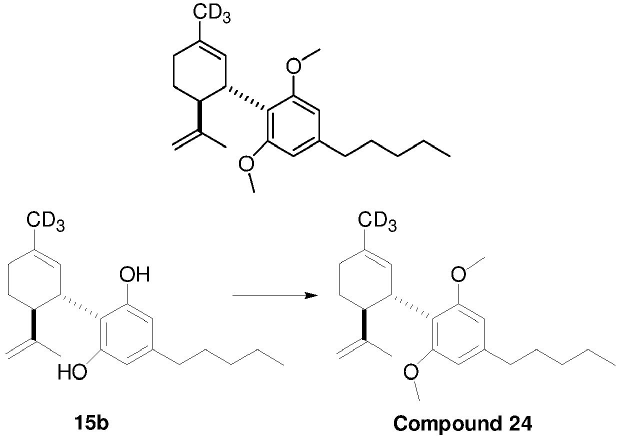

Compound 15b (1 mmol) was dissolved in DMF (55 ml), K₂CO₃ (5 mmol) and CH₃I (3.6 mmol) were added to the reaction mixture, and stirred at room temperature for 4 hours. The reaction was monitored by thin layer chromatography (10% ether/light petroleum ether) until the starting materials disappeared. 200ml of water was added to the reaction mixture, then the resultant mixture was extracted with ether. The resultant organic phases were washed with brine to neutral pH, dried over sodium sulfate, and the solvent was removed under reduced pressure. After purification by column chromatography, compound 24 was obtained (white solid, 342 mg, 99%).

LC-MS m/z (ESI) = 346.3 [M+1].

The following intermediates were synthesized according to the method indicated in the table.

| No. | preparation method | Structural formula | [M+H]+ |
|---|---|---|---|
| Intermediate 18-1 | With reference to J. Label. Compd. Radiopharm. 2002, 45, 1065-1076, 1.8g of Intermediate 18-1 was prepared. | 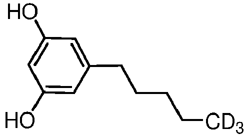 | - |
| Intermediate 18 | Compound 13b and intermediate 18-1 were used as raw materials, and intermediate 18 was prepared with reference to the fourth step in the synthesis method of Example 1. | 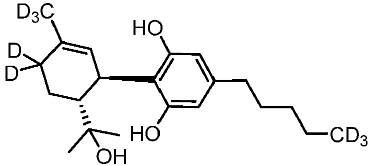 | 341.3 |
| Intermediate 19-1 | With reference to J. Label. Compd. Radiopharm. 2002, 45, 1065-1076, 3.1 g of Intermediate 19-1 was prepared. | 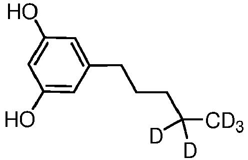 | - |
| Intermediate 19 | Compound 13b and intermediate 19-1 were used as raw materials, and intermediate 19 was prepared with reference to the fourth step in the synthesis method of Example 1. | 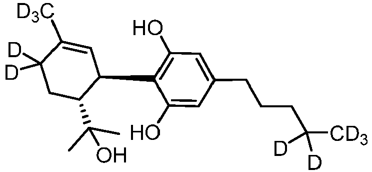 | 343.3 |
| Intermediate 20-1 | With reference to J. Label. Compd. Radiopharm. 2002, 45, 1065-1076, 1.3 g of Intermediate 20-1 was prepared. | 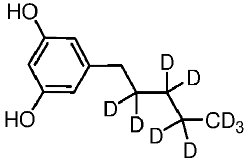 | - |
| Intermediate 20 | Compound 13b and intermediate 20-1 were used as raw materials, and intermediate 20 was prepared with reference to the fourth step in the synthesis method of Example 1. | 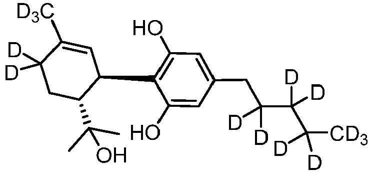 | 348.3 |
| Intermediate 23-1 | Commercially available (cas: 491-72-5) | 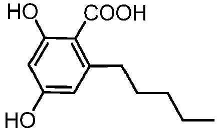 | |
| Intermediate 23 | Compound 1d and intermediate 23-1 were used as raw materials, and intermediate 23 was prepared with reference to the fourth step in the synthesis method of Example 1. | 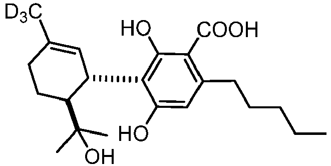 | 380.2 |
| Intermediate 25-1 | 5.1g of intermediate 25-1 was prepared with reference to WO2019232413. | 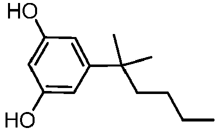 | - |
| Intermediate 25 | Compound 1d and intermediate 25-1 were used as raw materials, and intermediate 25 was prepared with reference to the fourth step in the synthesis method of Example 1. | 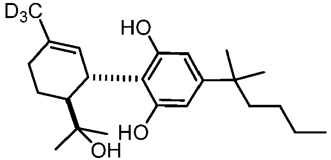 | 364.3 |
| Intermediate 26-1 | Intermediate 26-1 was prepared with reference to WO2014134127. | 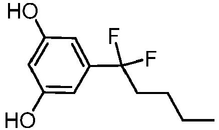 | - |
| Intermediate 26 | Compound 1d and intermediate 26-1 were used as raw materials, and intermediate 26 was prepared with reference to the fourth step in the synthesis method of Example 1. | 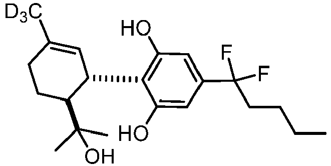 | 372.2 |
| Intermediate 27-1 | With reference to J. Med. Chem. 2009, 52, 2506-2514, 3.9 g of Intermediate 27-1 was prepared. | 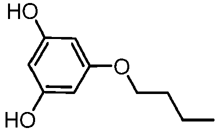 | - |
| Intermediate 27 | Compound 1d and intermediate 27-1 were used as raw materials, and intermediate 27 was prepared with reference to the fourth step in the synthesis method of Example 1. | 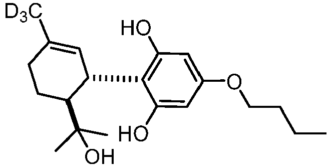 | 338.2 |
| Intermediate 28-1 | With reference to Bioorg. Med. Chem. Lett. 2002, 12, 3583-3586, 1.1 g of Intermediate 28-1 was prepared. | 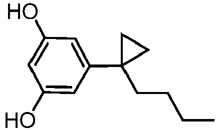 | - |
| Intermediate 28 | Compound 1d and intermediate 28-1 were used as raw materials, and intermediate 28 was prepared with reference to the fourth step in the synthesis method of Example 1. | 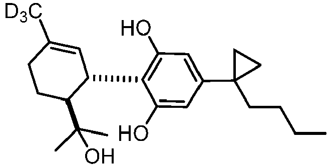 | 362.3 |
| Intermediate 29-1 | With reference to Org. Prep. & Proc. Int. 1979, 11, 87-92, 872 mg of Intermediate 29-1 was prepared. | 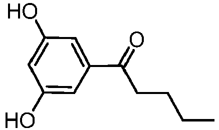 | |
| Intermediate 29 | Compound 1d and intermediate 29-1 were used as raw materials, and intermediate 29 was prepared with reference to the fourth step in the synthesis method of Example 1. | 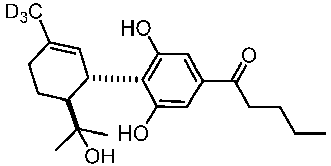 | 350.2 |

The following compounds were synthesized according to the method indicated in the table.

| No. | preparation method | Structural formula | [M+H]+ |
|---|---|---|---|
| Example 16 | Compound 13 was used as raw materials, and compound 16 was prepared with reference to the third step in the synthesis method of Example 15. | 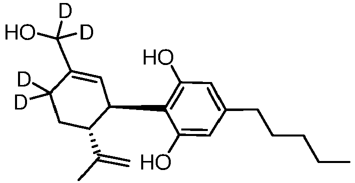 | 335.2 |
| Example 17 | Compound 18 was used as raw materials, and compound 17 was prepared with reference to the third step in the synthesis method of Example 15. | 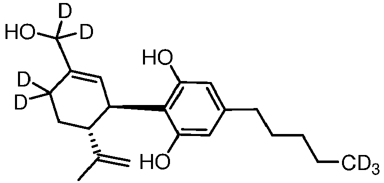 | 338.2 |
| Example 18 | Intermediate 18 was used as raw materials, and compound 18 was prepared with reference to the fifth step in the synthesis method of Example 1. | 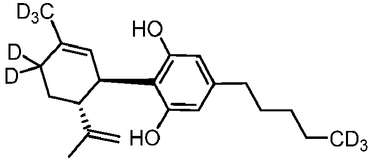 | 323.3 |
| Example 19 | Intermediate 19 was used as raw materials, and compound 19 was prepared with reference to the fifth step in the synthesis method of Example 1. | 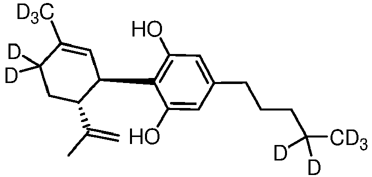 | 325.3 |
| Example 20 | Intermediate 20 was used as raw materials, and compound 20 was prepared with reference to the fifth step in the synthesis method of Example 1. | 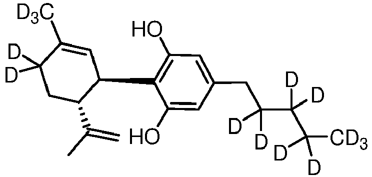 | 331.3 |
| Example 21 | Compound 15b was used as raw materials, and 71mg of compound 21 was prepared with reference to WO2017008136. | 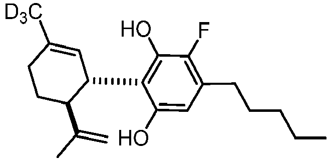 | 336.2 |
| Example 23 | Intermediate 23 was used as raw materials, and compound 23 was prepared with reference to the fifth step in the synthesis method of Example 1. | 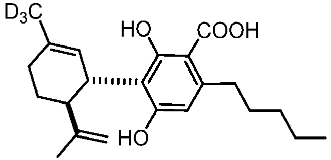 | 362.2 |
| Example 25 | Intermediate 25 was used as raw materials, and compound 25 was prepared with reference to the fifth step in the synthesis method of Example 1. | 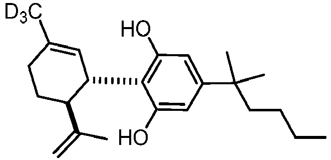 | 346.3 |
| Example 26 | Intermediate 26 was used as raw materials, and compound 26 was prepared with reference to the fifth step in the synthesis method of Example 1. | 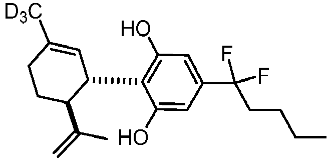 | 354.2 |
| Example 27 | Intermediate 27 was used as raw materials, and compound 27 was prepared with reference to the fifth step in the synthesis method of Example 1. | 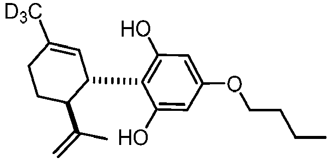 | 320.2 |
| Example 28 | Intermediate 28 was used as raw materials, and compound 28 was prepared with reference to the fifth step in the synthesis method of Example 1. | 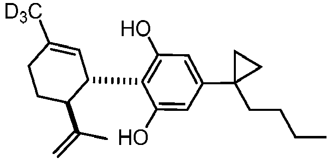 | 344.3 |
| Example 29 | Intermediate 29 was used as raw materials, and compound 29 was prepared with reference to the fifth step in the synthesis method of Example 1. | 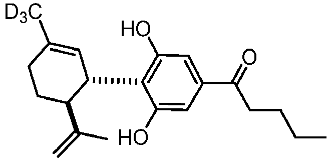 | 332.3 |
| Example 30 | Compound 22 was used as raw materials, and compound 30 was prepared with reference to the synthesis method of Example 24. | 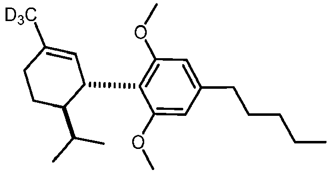 | 346.3 |

### Pharmacokinetics in rats

Healthy adult SD rats (n = 3 per group) were fasted overnight (free access to water), and then were administrated the drugs by intragastric administration (p.o.) (50mg/kg). 0.1 mL of blood was collected from the jugular venous plexus of the rats at 15 minutes, 30 minutes, 1 hour, 2 hours, 4 hours, 6 hours, 8 hours, 24 hours after administration. All blood samples were anticoagulated with K₂EDTA, then centrifuged at 3500rpm at 5°C for 10minutes to separate plasma, and stored at -20°C for test. Parent drug concentration in plasma was determined by LC/MS/MS method. The pharmacokinetic parameters of the compound after intragastric administration (p.o.) in rats were measured, and the results are as follows:

| No. | Tₘₐₓ (h) | Cₘₐₓ (ng/mL) | AUC₀₋ₜ (ng·h/mL) | AUC_{0-∞} (ng·h/mL) | t_{1/2} |
|---|---|---|---|---|---|
| CBD | 2.0 | 784 | 4463 | 4497 | 4.8 |
| Example 3 | 2.1 | 795 | 4229 | 4481 | 4.9 |
| Example 7 | 2.2 | 901 | 5829 | 6291 | 6.5 |
| Example 9 | 2.0 | 823 | 6217 | 6483 | 7.9 |
| Example 13 | 2.0 | 871 | 5979 | 6178 | 6.0 |
| Example 16 | 1.5 | 860 | 5494 | 5628 | 5.8 |
| Example 23 | 1.75 | 977 | 6982 | 8226 | 6.5 |

Experiment results showed that the compounds of the present application show better pharmacokinetic characteristics and better oral bioavailability than CBD after being orally administered to rats.

Although the specific embodiments are described in detail in the description of the present application, those skilled in the art should understand that the above embodiments are exemplary and cannot be understood as a limitation to the present application. If those skilled in the art make improvements and modifications to the present application without departing from the principle of the present application, the technical solution obtained by these improvements and modifications shall also fall within the protection scope of the claims of the present application.

## Claims

1. A compound represented by general formula (I), or stereoisomers, solvates, prodrugs, metabolites, pharmaceutically acceptable salts or cocrystals thereof; 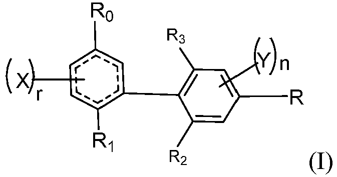 wherein,
R₀ is selected from the group consisting of methyl, C₃₋₈ carbocyclic group, -CH₂OH, - C(=O)OC₁₋₆ alkyl, -C(=O)NR^{b1}R^{b2} and carboxyl, and at least one hydrogen atom of R₀ is substituted by deuterium atom, and when R₀ is selected from the group consisting of -CD₂H and -CD₃, R is not -(CH₂)₄CH₃;
X is selected from the group consisting of hydrogen, deuterium, hydroxyl, C₁₋₆ alkyl and halogen;
R₁ is selected from the group consisting of C₁₋₆ alkyl, C₃₋₈ carbocyclic group and C₂₋₆ alkenyl, wherein the C₁₋₆ alkyl, the C₂₋₆ alkenyl or the C₃₋₈ carbocyclic group is optionally substituted by 1 to 3 substituents selected from the group consisting of halogen, hydroxyl, C₃₋₈ carbocyclic group and C₁₋₆ alkyl, and R₁ is optionally substituted by one or more deuterium atoms;
R₂ and R₃ are each independently selected from the group consisting of hydrogen, hydroxyl and C₁₋₆ alkoxy, R₂ and R₃ are each independently optionally substituted by one or more deuterium atoms, and at least one of R₂ and R₃ is not H;
r is selected from 0, 1, 2 and 3;
n is selected from 0, 1 and 2;
Y is selected from the group consisting of hydrogen, carboxyl, C₁₋₆ alkyl and halogen, and Y is optionally substituted by one or more deuterium atoms;
R is selected from the group consisting of C₁₋₁₂ alkyl, C₁₋₁₂ heteroalkyl, C₂₋₁₂ alkenyl, C₂₋₁₂ alkynyl, C₃₋₁₂ carbocyclic group, C₃₋₁₂ heterocyclic group, -C₁₋₆ alkylene-C₃₋₁₂ carbocyclic group, -C₁₋₆ alkylene-C₃₋₁₂ heterocyclic group, -NR^{b1}R^{b2}, -C₁₋₆ alkylene-C(=O)OC₁₋₆ alkyl and -C₁₋₆ alkylene-C(=O)NR^{b1}R^{b2}, and the C₁₋₁₂ alkyl, the C₁₋₁₂ heteroaklyl, the C₂₋₁₂ alkenyl, the C₂₋₁₂ alkynyl, the C₁₋₆ alkylene, the C₃₋₁₂ carbocyclic group and the C₃₋₁₂ heterocyclic group are optionally substituted with one or more substituents selected from the group consisting of hydroxyl, carboxyl, halogen, cyano, =O, C₁₋₆ alkyl, -NR^{b1}R^{b2}, C₃₋₁₂ carbocyclic group, C₃₋₁₂ heterocyclic group, C₂₋₆ alkenyl, C₂₋₆ alkynyl, -C(=O)OC₁₋₆ alkyl, -C(=O)C₁₋₆ alkyl, - C(=O)NR^{b1}R^{b2}, -S(=O)C₁₋₆ alkyl and -S(=O)₂C₁₋₆ alkyl, wherein, as substituents, the C₁₋₆ alkyl, the C₃₋₁₂ carbocyclic group and the C₃₋₁₂ heteracyclic group are optionally further substituted with one or more substituents selected from the group consisting of =O, hydroxyl, carboxyl, halogen, cyano, -C(=O)OC₁₋₆ alkyl and -C(=O)C₁₋₆ alkyl, R is optionally substituted by one or more deuterium atoms;
R^{b1} and R^{b2} are each independently selected from the group consisting of H, C₁₋₆ alkyl, C₃₋₁₂ carbocyclic group, C₃₋₁₂ heterocyclic group, -C(=O)R^{b3} and -C(=O)NR^{b4}R^{b5}, wherein the C₁₋₆ alkyl, C₃₋₁₂ carbocyclic group and C₃₋₁₂ heterocyclic group are optionally further substituted by one or more groups selected from the group consisting of hydroxy, deuterium atom, halogen, C₁₋₆ alkyl, C₁₋₆ alkoxy, C₆₋₁₂ aryl, C₅₋₁₂ hetroaryl, C₃₋₁₂ cycloalkyl, and C₃₋₁₂ heterocycloalkyl; or R^{b4} and R^{b5} together with N atom form a 3 to 12 membered heterocycle containing one or more heteroatoms selected from the group consisting of N, O and S, and R^{b1} and R^{b2} are optionally substituted by one or more deuterium atoms;
R^{b3} is selected from the group consisting of C₁₋₆ alkyl, C₁₋₆ alkoxy and C₆₋₁₂ aryl; and R^{b3} is optionally substituted by one or more deuterium atoms;
R^{b4} and R^{b5} are selected from the group consisting of H and C₁₋₆ alkyl, and R^{b4} and R^{b5} are optionally substituted by one or more deuterium atoms; 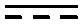
is a single bond or a double bond.

2. The compound, or stereoisomers, solvates, prodrugs, metabolites, pharmaceutically acceptable salts or cocrystals thereof according to claim 1, wherein the compound is selected from the group consisting of:

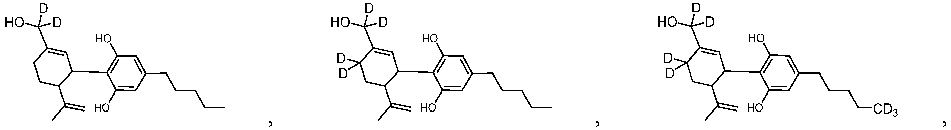

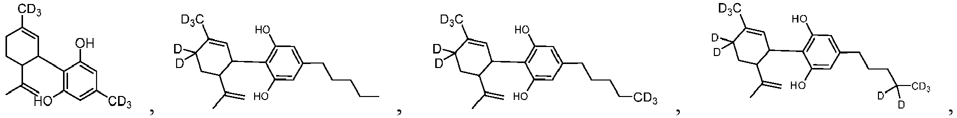

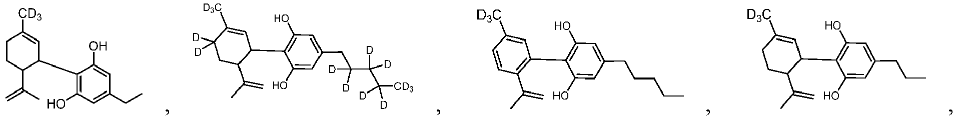

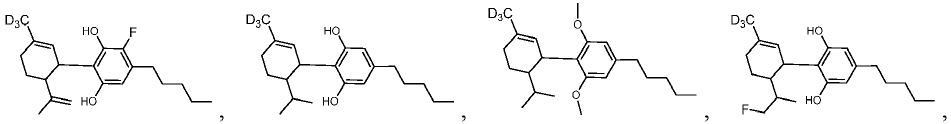

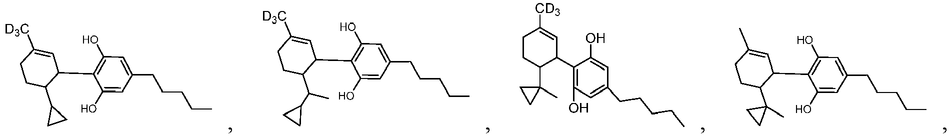

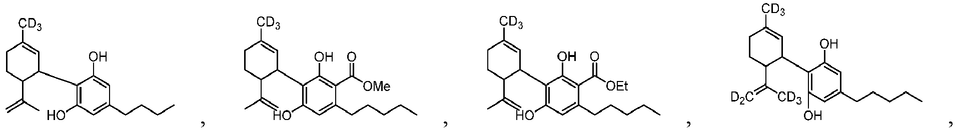

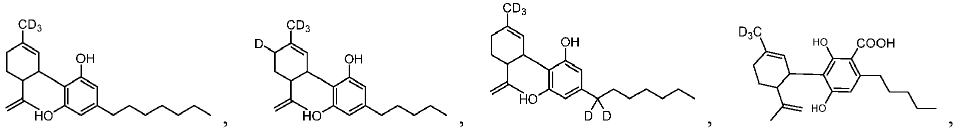

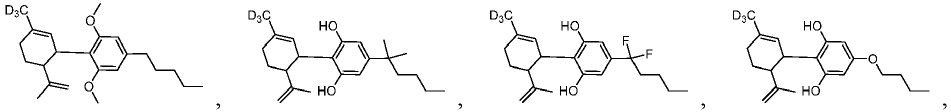

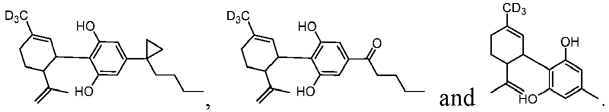

3. A intermediate for preparing the compound represented by general formula (I), or stereoisomers, solvates, prodrugs, metabolites, pharmaceutically acceptable salts or cocrystals thereof, wherein the intermediate is selected from the group consisting of: 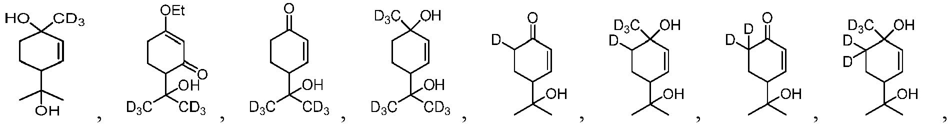 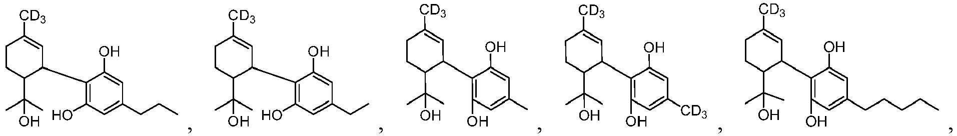 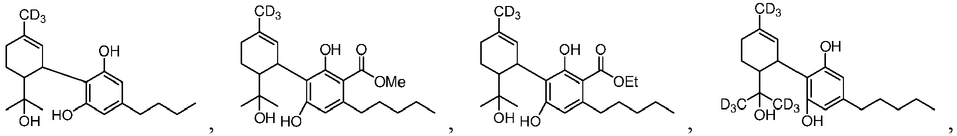 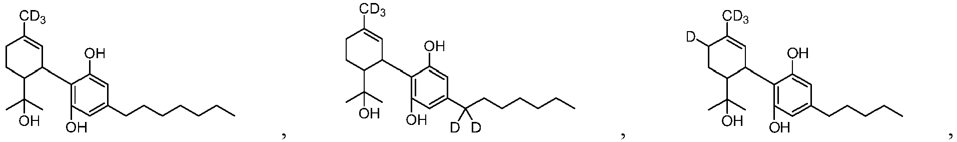 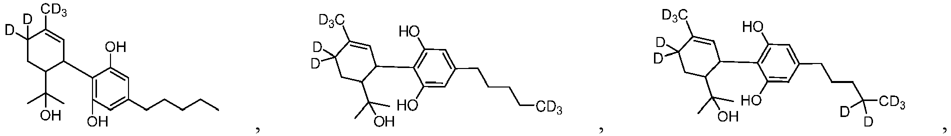 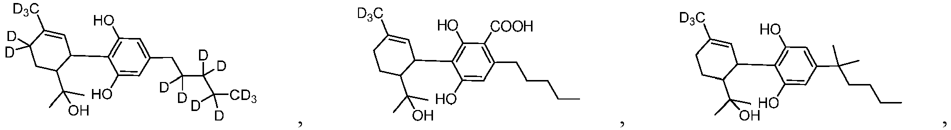 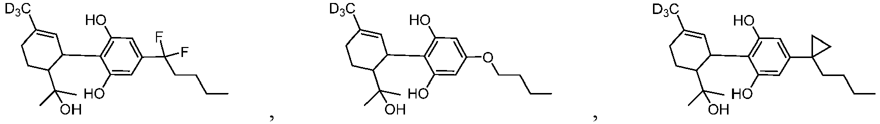 and

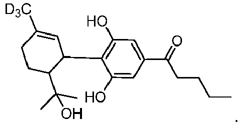

4. A method for preparing compound A or stereoisomers, deuterated products, solvates, prodrugs, metabolites, pharmaceutically acceptable salts or cocrystals thereof, comprising steps of: 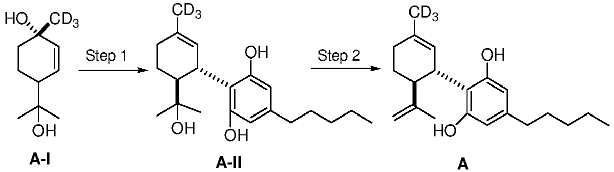
Step 1: reacting compound A-I, 3,5-dihydroxyamylbenzene and a protonic acid in an organic solvent to obtain compound A-II;
Step 2: subjecting compound A-II to a dehydration reaction in an organic solvent to obtain compound A.

5. A pharmaceutical composition comprising:
(1) the compound, or stereoisomers, solvates, metabolites, pharmaceutically acceptable salts, cocrystals or prodrugs thereof according to any one of claims 1 to 3,
(2) optional one or more other active ingredients; and
(3) a pharmaceutically acceptable carrier and/or excipient.

6. The pharmaceutical composition according to claim 5, wherein, the other active ingredient is one or more selected from the group consisting of ginkgolides, antineoplastic agents, anticoagulants, antiepileptic agents, antidepressants, anxiolytics, hypnotics, analgesics or anesthetics, or stereoisomers, hydrates, metabolites, solvates, pharmaceutically acceptable salts and cocrystals of the other active ingredients; preferably, the ginkgolides are one selected from the group consisting of ginkgolide A, ginkgolide B, ginkgolide C, ginkgolide D, ginkgolide J, ginkgolide M, ginkgolide K, ginkgolide L, ginkgolide N, ginkgolide P, ginkgolide Q and bilobalide or combinations of two or more thereof in any ratio.

7. Use of the compound, or stereoisomers, solvates, prodrugs, metabolites, pharmaceutically acceptable salts or cocrystals thereof according to claim 1 or 2, or the pharmaceutical composition according to claim 5 or 6 in the preparation of a medicament for treating post-traumatic stress disorder, facial paralysis, stroke, migraine, coronary heart disease stable angina pectoris, cerebral infarction, thromboembolism, myocardial infarction, cardiac ischemia, coronary artery disease, hypertension, cerebral ischemia, sexual function improvement, spasm, acute and chronic pain, fibromyalgia, postoperative pain, cluster headache, tension headache, back pain, limb pain, osphyalgia, neck pain, neuropathic pain, cancer pain, trigeminal neuralgia, arthritic pain, inflammatory pain, Dravet syndrome, Lennox-Gastaut syndrome, Prader-Willi syndrome, Sturge-Weber syndrome, fragile X syndrome, anxiety, bipolar affective disorder, autism, general anxiety disorder, social anxiety disorder, epilepsy, Parkinson's disease, Alzheimer's disease, Huntington's disease, opioid abuse, alcoholism, nicotine addiction, anorexia, cachexia, chemotherapy-related nausea and vomiting, postoperative nausea and vomiting, amyotrophic lateral sclerosis (ALS), Friedreich ataxia, schizophrenia, obsessive-compulsive disorder, multiple sclerosis, depression, sleep disorder, spasm caused by multiple sclerosis, dysmyotonia, sleep apnea, paralytic dementia, hypomnesis or glioblastoma.
